# EUROPEAN PATENT APPLICATION

(11) **EP 4 793 362 A1**
(43) Date of publication of application: **19.08.2026**
(21) Application number: 24877160.2
(22) Date of filing: 07.10.2024
(51) Int. Cl.: C12Q 1/6806, C12N 15/00, C12N 15/11, C40B 40/06

(54) **LIGATION METHOD, METHOD FOR INHIBITING FORMATION OF ADAPTER DIMER, METHOD FOR PREPARING NGS LIBRARY, KIT FOR PREPARING NGS LIBRARY, AND 3' ADAPTER**

(30) Priority: 13.10.2023 JP 2023177843; 25.04.2024 JP 2024071785
(71) Applicant: ARKRAY, Inc., Kyoto-shi, Kyoto 601-8045 (JP)
(72) Inventor: HOJO, Hiroaki, Kyoto-shi, Kyoto 602-0008 (JP); NISHIDA, Ruisa, Kyoto-shi, Kyoto 602-0008 (JP)
(74) Representative: MFG Patentanwälte Meyer-Wildhagen Meggle-Freund Gerhard PartG mbB
(86) International application number: PCT/JP2024/035868
(87) International publication number: WO 2025/079565

(57) **Abstract**

A ligation method including: ligating, to a 3' end of each of plural kinds of nucleic acids having different sequences, a 5'-adenylated single-stranded DNA that is a 3' adapter in which a base sequence of a 5' end excluding an adenylic acid at the 5' end is any one of base sequences shown in Table A; and after the ligating of the 5'-adenylated single-stranded DNA, ligating, to a 5' end of each of the plural kinds of nucleic acids, a single-stranded nucleic acid that is a 5' adapter; a method of suppressing adapter dimer formation; a method of preparing an NGS library; an NGS library preparation kit; and a 3' adapter.

## Description

### Technical Field

The present disclosure relates to a ligation method, a method of suppressing adapter dimer formation, a method of preparing an NGS library, an NGS library preparation kit, and a 3' adapter.

### Background Art

In recent years, exhaustive quantitative analysis of nucleic acids represented by Next-Generation Sequencing (hereinafter also referred to as NGS) has been applied to the discrimination of diseases and the like. In library preparation for NGS, base sequences called a 5' adapter and a 3' adapter are first added by ligation to the 5' end and the 3' end, respectively, of a target base sequence to be analyzed. When the nucleic acid to be analyzed is RNA, the target base sequence with the adapters added to both ends is subsequently converted into cDNA by a reverse transcription reaction. Thereafter, DNA is amplified by a polymerase chain reaction (hereinafter also referred to as PCR) using a primer having a sequence necessary for sequence analysis (see, e.g., Non-Patent Literature 1).

In the library preparation, ligation requires strict condition setting. The type of ligation enzyme and the sequence of the adapter nucleic acid affect the success or failure of the ligation reaction. For example, it is known that ligase activity may vary depending on the batch (see Non-Patent Literature 2). In addition, regarding the influence of the sequence of the adapter nucleic acid, it is known that the quantitative bias against the RNA species to be measured can be reduced by randomizing its terminal sequence (see, e.g., Patent Literature 1 and Non-Patent Literature 3). More specifically, in Non-Patent Literature 3, 3' adapters in which the base sequence of the 5' end is randomized are ligated to RNAs in the presence of a high concentration of polyethylene glycol (PEG). Then, after the ligation, surplus 3' adapters that were not ligated are removed using a polyacrylamide gel, and the RNAs to which the 3' adapters are added are purified. Then, the purified RNAs to which the 3' adapters are added are further ligated to 5' adapters in which the base sequence of the 3' end is randomized, in the presence of a high concentration of PEG.
Patent Literature 1: US Patent No. 9487825
Non-Patent Literature 1: Seda Eminaga et al. Quantification of microRNA expression with next-generation sequencing. Curr Protoc Mol Biol. 2013 July: Chapter 4: Unit 4.17
Non-Patent Literature 2: Andreas Mayer; L Stirling Churchman, Genome-wide profiling of RNA polymerase transcription at nucleotide resolution in human cells with native elongating transcript sequencing. Nat Protoc. 2016 Apr;11(4):813-33
Non-Patent Literature 3: Haedong Kim et al. Bias-minimized quantification of microRNA reveals widespread alternative processing and 3' end modification. Nucleic Acids Res. 2019 Mar 18;47(5):2630-2640

### SUMMARY OF INVENTION

### Technical Problem

In the above ligation operation, a side reaction also occurs in which surplus 5' adapters and surplus 3' adapters that were not ligated to the target base sequence bind to each other to generate adapter dimers. Since PCR of the adapter dimers also occurs along with PCR of the target base sequence, sequence reads derived from the adapter dimers are also obtained in subsequent sequencing. This decreases sequencing efficiency (i.e., the number of reads per amount of data). In Non-Patent Literature 3, surplus 3' adapters are removed, and the RNAs to which the 3' adapters are added are purified, and then the ligation of the 5' adapters is performed. However, a method that can improve sequencing efficiency in a simpler manner is desired.

In view of such a situation, the present disclosure relates to a ligation method, a method of suppressing adapter dimer formation, a method of preparing an NGS library, an NGS library preparation kit, and a 3' adapter that can improve sequencing efficiency in a simpler manner.

### Solution to Problem

Means for solving the foregoing problem include the following aspects.
(1) A ligation method, including:
   ligating, to a 3' end of each of a plurality of kinds of nucleic acids having different sequences, a 5'-adenylated single-stranded DNA that is a 3' adapter in which a base sequence of a 5' end excluding an adenylic acid at the 5' end is any one of base sequences shown in the following Table A; and
   after the ligating of the 5'-adenylated single-stranded DNA, ligating, to a 5' end of each of the plurality of kinds of nucleic acids, a single-stranded nucleic acid that is a 5' adapter.

**[Table 1]**

| Table A | | | | | | | |
|---|---|---|---|---|---|---|---|
| Number | Base sequence (5'→3') | Number | Base sequence (5'→3') | Number | Base sequence (5'→3') | Number | Base sequence (5'→3') |
| 1 | AAAA | 21 | GAGA | 41 | TCGA | 61 | GGAC |
| 2 | AAAC | 22 | GGAA | 42 | TCTC | 62 | GGCA |
| 3 | AAAG | 23 | GGAG | 43 | TCTT | 63 | GGCT |
| 4 | AAAT | 24 | GGGA | 44 | TGAA | 64 | GGGG |
| 5 | AACA | 25 | GTCG | 45 | TGAG | 65 | GTAG |
| 6 | AACG | 26 | TAAA | 46 | TGCA | 66 | TCAC |
| 7 | AACT | 27 | TAAC | 47 | TGGA | 67 | TGAC |
| 8 | AAGA | 28 | TAAG | 48 | TGGG | 68 | TGCG |
| 9 | AATT | 29 | TACA | 49 | TGTA | 69 | TGCT |
| 10 | ACAA | 30 | TACC | 50 | TTCT | 70 | TGGC |
| 11 | AGAA | 31 | TACG | 51 | TTTA | 71 | TTAT |
| 12 | AGAG | 32 | TAGA | 52 | TTTC | 72 | GCAA |
| 13 | AGCA | 33 | TAGG | 53 | TTTT | 73 | GCAG |
| 14 | AGCG | 34 | TATT | 54 | AACC | 74 | GTAA |
| 15 | AGCT | 35 | TCAA | 55 | AAGG | 75 | GTCA |
| 16 | AGGA | 36 | TCAG | 56 | ACTT | 76 | TCGG |
| 17 | AGGC | 37 | TCCA | 57 | AGTT | 77 | TCTA |
| 18 | AGGG | 38 | TCCC | 58 | GAAG | 78 | AGAT |
| 19 | AGTA | 39 | TCCG | 59 | GACA | | |
| 20 | GAAA | 40 | TCCT | 60 | GACC | | |

(2) The ligation method according to (1), wherein a ligase used for the ligation of the 3' adapter and a ligase used for the ligation of the 5' adapter are each independently at least one selected from the group consisting of T4 RNA Ligase 1, T4 RNA Ligase 2, T4 DNA ligase, and a variant thereof.

(3) The ligation method according to (1) or (2), wherein the base sequence of the 5' end of the 3' adapter is any one of base sequences shown in the following Table B.

**[Table 2]**

| Table B | | |
|---|---|---|
| GACA | GTCA | AACT |
| ACAA | TTAT | AGGA |
| AACA | TGAA | AACG |

(4) The ligation method according to any one of (1) to (3), wherein a ligase used for the ligation of the 3' adapter is T4 RNA Ligase 1.

(5) The ligation method according to any one of (1) to (4), wherein each of the plurality of kinds of nucleic acids is a small RNA.

(6) A method of suppressing adapter dimer formation, the method including:
ligating, to a 3' end of each of a plurality of kinds of nucleic acids having different sequences, a 5'-adenylated single-stranded DNA that is a 3' adapter in which a base sequence of a 5' end, excluding an adenylic acid at the 5' end, is any one of base sequences shown in the following Table A; and
after the ligating of the 5'-adenylated single-stranded DNA, ligating, to a 5' end of each of the plurality of kinds of nucleic acids, a single-stranded nucleic acid that is a 5' adapter.

**[Table 3]**

| Table A | | | | | | | |
|---|---|---|---|---|---|---|---|
| Number | Base sequence (5'→3') | Number | Base sequence (5'→3') | Number | Base sequence (5'→3') | Number | Base sequence (5'→3') |
| 1 | AAAA | 21 | GAGA | 41 | TCGA | 61 | GGAC |
| 2 | AAAC | 22 | GGAA | 42 | TCTC | 62 | GGCA |
| 3 | AAAG | 23 | GGAG | 43 | TCTT | 63 | GGCT |
| 4 | AAAT | 24 | GGGA | 44 | TGAA | 64 | GGGG |
| 5 | AACA | 25 | GTCG | 45 | TGAG | 65 | GTAG |
| 6 | AACG | 26 | TAAA | 46 | TGCA | 66 | TCAC |
| 7 | AACT | 27 | TAAC | 47 | TGGA | 67 | TGAC |
| 8 | AAGA | 28 | TAAG | 48 | TGGG | 68 | TGCG |
| 9 | AATT | 29 | TACA | 49 | TGTA | 69 | TGCT |
| 10 | ACAA | 30 | TACC | 50 | TTCT | 70 | TGGC |
| 11 | AGAA | 31 | TACG | 51 | TTTA | 71 | TTAT |
| 12 | AGAG | 32 | TAGA | 52 | TTTC | 72 | GCAA |
| 13 | AGCA | 33 | TAGG | 53 | TTTT | 73 | GCAG |
| 14 | AGCG | 34 | TATT | 54 | AACC | 74 | GTAA |
| 15 | AGCT | 35 | TCAA | 55 | AAGG | 75 | GTCA |
| 16 | AGGA | 36 | TCAG | 56 | ACTT | 76 | TCGG |
| 17 | AGGC | 37 | TCCA | 57 | AGTT | 77 | TCTA |
| 18 | AGGG | 38 | TCCC | 58 | GAAG | 78 | AGAT |
| 19 | AGTA | 39 | TCCG | 59 | GACA | | |
| 20 | GAAA | 40 | TCCT | 60 | GACC | | |

(7) The method of suppressing adapter dimer formation according to (6), wherein a ligase used for the ligation of the 3' adapter and a ligase used for the ligation of the 5' adapter are each independently at least one selected from the group consisting of T4 RNA Ligase 1, T4 RNA Ligase 2, T4 DNA ligase, and a variant thereof.

(8) A method of preparing an NGS library, the method including:
ligating, to a plurality of kinds of nucleic acids having different sequences, a 5'-adenylated single-stranded DNA that is a 3' adapter and a single-stranded nucleic acid that is a 5' adapter by the ligation method according to any one of (1) to (5);
synthesizing a cDNA by a reverse transcription reaction from the plurality of kinds of nucleic acids to which the 3' adapter and the 5' adapter have been ligated; and
amplifying the obtained cDNA by PCR.

(9) The method of preparing an NGS library according to (8), wherein the plurality of kinds of nucleic acids having different sequences are a plurality of kinds of RNAs having different sequences.

(10) An NGS library preparation kit, including a 5'-adenylated single-stranded DNA contained in a first container, wherein a base sequence of a 5' end of the 5'-adenylated single-stranded DNA, excluding an adenylic acid at the 5' end, is any one of base sequences shown in the following Table A.

**[Table 4]**

| Table A | | | | | | | |
|---|---|---|---|---|---|---|---|
| Number | Base sequence (5'→3') | Number | Base sequence (5'→3') | Number | Base sequence (5'→3') | Number | Base sequence (5'→3') |
| 1 | AAAA | 21 | GAGA | 41 | TCGA | 61 | GGAC |
| 2 | AAAC | 22 | GGAA | 42 | TCTC | 62 | GGCA |
| 3 | AAAG | 23 | GGAG | 43 | TCTT | 63 | GGCT |
| 4 | AAAT | 24 | GGGA | 44 | TGAA | 64 | GGGG |
| 5 | AACA | 25 | GTCG | 45 | TGAG | 65 | GTAG |
| 6 | AACG | 26 | TAAA | 46 | TGCA | 66 | TCAC |
| 7 | AACT | 27 | TAAC | 47 | TGGA | 67 | TGAC |
| 8 | AAGA | 28 | TAAG | 48 | TGGG | 68 | TGCG |
| 9 | AATT | 29 | TACA | 49 | TGTA | 69 | TGCT |
| 10 | ACAA | 30 | TACC | 50 | TTCT | 70 | TGGC |
| 11 | AGAA | 31 | TACG | 51 | TTTA | 71 | TTAT |
| 12 | AGAG | 32 | TAGA | 52 | TTTC | 72 | GCAA |
| 13 | AGCA | 33 | TAGG | 53 | TTTT | 73 | GCAG |
| 14 | AGCG | 34 | TATT | 54 | AACC | 74 | GTAA |
| 15 | AGCT | 35 | TCAA | 55 | AAGG | 75 | GTCA |
| 16 | AGGA | 36 | TCAG | 56 | ACTT | 76 | TCGG |
| 17 | AGGC | 37 | TCCA | 57 | AGTT | 77 | TCTA |
| 18 | AGGG | 38 | TCCC | 58 | GAAG | 78 | AGAT |
| 19 | AGTA | 39 | TCCG | 59 | GACA | | |
| 20 | GAAA | 40 | TCCT | 60 | GACC | | |

(11) The NGS library preparation kit according to (10), wherein the base sequence of the 5' end of the single-stranded DNA, excluding the adenylic acid at the 5' end, is any one of base sequences shown in the following Table B.

**[Table 5]**

| Table B | | |
|---|---|---|
| GACA | GTCA | AACT |
| ACAA | TTAT | AGGA |
| AACA | TGAA | AACG |

(12) The NGS library preparation kit according to (10) or (11), further including T4 RNA Ligase 1 contained in a second container.

(13) The NGS library preparation kit according to any one of (10) to (12), further including at least one selected from the group consisting of a 5' adapter, a ligase, a reverse transcriptase, a reverse transcription primer, a PCR enzyme, and a PCR primer, in a manner in which each of these is contained in an individual container.

(14) A 3' adapter for preparing a nucleic acid library, which is a single-stranded DNA in which a nucleotide at a 5' end is adenylated, wherein a base sequence of the 5' end of the single-stranded DNA, excluding an adenylic acid at the 5' end, is any one of base sequences shown in the following Table A.

**[Table 6]**

| Table A | | | | | | | |
|---|---|---|---|---|---|---|---|
| Number | Base sequence (5'→3') | Number | Base sequence (5'→3') | Number | Base sequence (5'→3') | Number | Base sequence (5'→3') |
| 1 | AAAA | 21 | GAGA | 41 | TCGA | 61 | GGAC |
| 2 | AAAC | 22 | GGAA | 42 | TCTC | 62 | GGCA |
| 3 | AAAG | 23 | GGAG | 43 | TCTT | 63 | GGCT |
| 4 | AAAT | 24 | GGGA | 44 | TGAA | 64 | GGGG |
| 5 | AACA | 25 | GTCG | 45 | TGAG | 65 | GTAG |
| 6 | AACG | 26 | TAAA | 46 | TGCA | 66 | TCAC |
| 7 | AACT | 27 | TAAC | 47 | TGGA | 67 | TGAC |
| 8 | AAGA | 28 | TAAG | 48 | TGGG | 68 | TGCG |
| 9 | AATT | 29 | TACA | 49 | TGTA | 69 | TGCT |
| 10 | ACAA | 30 | TACC | 50 | TTCT | 70 | TGGC |
| 11 | AGAA | 31 | TACG | 51 | TTTA | 71 | TTAT |
| 12 | AGAG | 32 | TAGA | 52 | TTTC | 72 | GCAA |
| 13 | AGCA | 33 | TAGG | 53 | TTTT | 73 | GCAG |
| 14 | AGCG | 34 | TATT | 54 | AACC | 74 | GTAA |
| 15 | AGCT | 35 | TCAA | 55 | AAGG | 75 | GTCA |
| 16 | AGGA | 36 | TCAG | 56 | ACTT | 76 | TCGG |
| 17 | AGGC | 37 | TCCA | 57 | AGTT | 77 | TCTA |
| 18 | AGGG | 38 | TCCC | 58 | GAAG | 78 | AGAT |
| 19 | AGTA | 39 | TCCG | 59 | GACA | | |
| 20 | GAAA | 40 | TCCT | 60 | GACC | | |

(15) The 3' adapter for preparing a nucleic acid library according to (14), wherein the base sequence of the 5' end of the single-stranded DNA, excluding the adenylic acid at the 5' end, is any one of base sequences shown in the following Table B.

**[Table 7]**

| Table B | | |
|---|---|---|
| GACA | GTCA | AACT |
| ACAA | TTAT | AGGA |
| AACA | TGAA | AACG |

(16) A 3' adapter for ligation, which is a single-stranded DNA in which a nucleotide at a 5' end is adenylated, wherein a base sequence of the 5' end of the single-stranded DNA, excluding an adenylic acid at the 5' end, is any one of base sequences shown in the following Table B.

**[Table 8]**

| Table B | | |
|---|---|---|
| GACA | GTCA | AACT |
| ACAA | TTAT | AGGA |
| AACA | TGAA | AACG |

### Advantageous Effects of Invention

According to the present disclosure, provided are a ligation method, a method of suppressing adapter dimer formation, a method of preparing an NGS library, an NGS library preparation kit, and a 3' adapter that can improve sequencing efficiency in a simpler manner.

### BRIEF DESCRIPTION OF DRAWINGS

Fig. 1 is a graph showing the correlation of the effective read rate of each 3' adapter between different kinds of 5' adapters in Example 1.
Fig. 2 shows the correlation of the quantitative values of miRNAs (average value of n=4) when two kinds of T4 RNA Ligase 1 are respectively used in the ligation of both the 3' adapter and the 5' adapter in Example 2.
Fig. 3 shows the correlation of the quantitative values of miRNAs (average value of n=4) when two kinds of T4 RNA Ligase 1 are used only in the ligation of the 3' adapter in Example 2.
Fig. 4 shows the correlation of the quantitative values of miRNAs (average value of n=4) when two kinds of T4 RNA Ligase 1 are used only in the ligation of the 5' adapter in Example 2.
Fig. 5 shows the correlation of the quantitative values of miRNAs (average value of n=2) when a 3' adapter in which the base sequence of the 5' end excluding the adenylic acid at the 5' end is TCCA is used and two kinds of T4 RNA Ligase 1 are used in Example 3.
Fig. 6 shows the correlation of the quantitative values of miRNAs (average value of n=2) when a 3' adapter in which the base sequence of the 5' end excluding the adenylic acid at the 5' end is GACA is used and two kinds of T4 RNA Ligase 1 are used in Example 3.

### DESCRIPTION OF EMBODIMENTS

Hereinafter, modes for carrying out the embodiments of the present disclosure will be described in detail. However, the embodiments of the present disclosure are not limited to the following embodiments. In the following embodiments, the constituent elements (including element steps and the like) are not essential unless otherwise explicitly stated. The same applies to numerical values and ranges, which do not limit the embodiments of the present disclosure.

In the present disclosure, the term "step" includes not only a step independent from other steps but also a step that cannot be clearly distinguished from other steps as long as the purpose of the step is achieved. In the present disclosure, a numerical range indicated using " to" includes the numerical values described before and after "to" as the minimum value and the maximum value, respectively.

In a numerical range described in a stepwise manner in the present disclosure, the upper limit value or the lower limit value described in one numerical range may be replaced with the upper limit value or the lower limit value of another numerical range described in a stepwise manner. Further, in a numerical range described in the present disclosure, the upper limit value or the lower limit value of the numerical range may be replaced with the value shown in the Examples.

In the present disclosure, each component may include multiple kinds of corresponding substances. When multiple kinds of substances corresponding to each component are present in the composition, the content or amount of each component means the total content or amount of the multiple kinds of substances present in the composition unless otherwise specified.

### <<Ligation Method>>

The ligation method according to the present disclosure includes:
ligating, to the 3' end of each of a plurality of kinds of nucleic acids having different sequences, a 5'-adenylated single-stranded DNA that is a 3' adapter in which the base sequence of the 5' end excluding the adenylic acid at the 5' end is any one of the base sequences shown in the following Table A; and,
after the ligating of the 5'-adenylated single-stranded DNA, ligating, to the 5' end of each of the plurality of kinds of nucleic acids, a single-stranded nucleic acid that is a 5' adapter.

**[Table 9]**

| Table A | | | | | | | |
|---|---|---|---|---|---|---|---|
| Number | Base sequence (5'→3') | Number | Base sequence (5'→3') | Number | Base sequence (5'→3') | Number | Base sequence (5'→3') |
| 1 | AAAA | 21 | GAGA | 41 | TCGA | 61 | GGAC |
| 2 | AAAC | 22 | GGAA | 42 | TCTC | 62 | GGCA |
| 3 | AAAG | 23 | GGAG | 43 | TCTT | 63 | GGCT |
| 4 | AAAT | 24 | GGGA | 44 | TGAA | 64 | GGGG |
| 5 | AACA | 25 | GTCG | 45 | TGAG | 65 | GTAG |
| 6 | AACG | 26 | TAAA | 46 | TGCA | 66 | TCAC |
| 7 | AACT | 27 | TAAC | 47 | TGGA | 67 | TGAC |
| 8 | AAGA | 28 | TAAG | 48 | TGGG | 68 | TGCG |
| 9 | AATT | 29 | TACA | 49 | TGTA | 69 | TGCT |
| 10 | ACAA | 30 | TACC | 50 | TTCT | 70 | TGGC |
| 11 | AGAA | 31 | TACG | 51 | TTTA | 71 | TTAT |
| 12 | AGAG | 32 | TAGA | 52 | TTTC | 72 | GCAA |
| 13 | AGCA | 33 | TAGG | 53 | TTTT | 73 | GCAG |
| 14 | AGCG | 34 | TATT | 54 | AACC | 74 | GTAA |
| 15 | AGCT | 35 | TCAA | 55 | AAGG | 75 | GTCA |
| 16 | AGGA | 36 | TCAG | 56 | ACTT | 76 | TCGG |
| 17 | AGGC | 37 | TCCA | 57 | AGTT | 77 | TCTA |
| 18 | AGGG | 38 | TCCC | 58 | GAAG | 78 | AGAT |
| 19 | AGTA | 39 | TCCG | 59 | GACA | | |
| 20 | GAAA | 40 | TCCT | 60 | GACC | | |

In the present disclosure, the "5'-adenylated single-stranded DNA that is a 3' adapter and in which the base sequence of the 5' end excluding the adenylic acid at the 5' end is any one of the base sequences shown in Table A" is also simply referred to as the "3' adapter according to the present disclosure". The "single-stranded nucleic acid that is a 5' adapter" is also simply referred to as the "5' adapter". The "plurality of kinds of nucleic acids having different sequences" indicates nucleic acids targeted for sequencing, and is also simply referred to as the "plurality of kinds of nucleic acids" or the "target nucleic acids". The base sequence of the 5' end of the 5'-adenylated single-stranded DNA excluding the adenylic acid at the 5' end means the base sequence of the 5' end assuming a non-adenylated single-stranded DNA.

The 3' adapter according to the present disclosure is easily ligated to the 3' end of each of the plurality of kinds of nucleic acids having different sequences. Although the reason why it is easily ligated is not clear, it is believed that the 3' adapter according to the present disclosure has high ligation efficiency to the respective 3' ends of the target nucleic acids, and thereby the sequencing efficiency is improved. This is believed to be partly due to the fact that, in the 3' adapter according to the present disclosure, the first base of the 5' end excluding the adenylic acid at the 5' end is adenine, thymine, or guanine.

Further, in the ligation method according to the present disclosure, after the ligation of the 3' adapter, a single-stranded nucleic acid that is a 5' adapter is ligated to the 5' end of each of the target nucleic acids.

Here, as described above, according to the conventional ligation method, a side reaction easily occurred in which surplus 5' adapters and surplus 3' adapters that were not ligated to the nucleic acids targeted for sequencing bound to each other to form adapter dimers.

On the other hand, according to the ligation method according to the present disclosure, a side reaction in which surplus 5' adapters and surplus 3' adapters that were not ligated to the nucleic acids targeted for sequencing bind to each other to form adapter dimers is less likely to occur. The reason why the adapter dimers are less likely to be formed is that the 3' adapter according to the present disclosure is unlikely to be ligated and/or hybridized with the 5' adapter. More specifically, when the sequence of the 4 bases at the 5' end of the 3' adapter excluding the adenylic acid at the 5' end is any one of the specific base sequences shown in Table A above, the 3' adapter is unlikely to be ligated and/or hybridized with the 5' adapter. This is believed to be partly due to the fact that, in the 3' adapter according to the present disclosure, the first base of the 5' end excluding the adenylic acid at the 5' end is adenine, thymine, or guanine.

As described above, the 3' adapter according to the present disclosure is easily ligated to the target nucleic acids, and furthermore, the 3' adapter according to the present disclosure is unlikely to form adapter dimers with the 5' adapter. For these reasons, it is believed that, according to the ligation method according to the present disclosure, sequencing efficiency can be improved in a simpler manner.

It is noted that the present disclosure is not limited to the above-described estimated mechanism in any way.

### <Ligation of 3' Adapter>

The ligation method according to the present disclosure includes ligating, to the 3' end of each of a plurality of kinds of nucleic acids, a 5'-adenylated single-stranded DNA that is a 3' adapter in which the base sequence of the 5' end excluding the adenylic acid at the 5' end is any one of the base sequences shown in the following Table A.

**[Table 10]**

| Table A | | | | | | | |
|---|---|---|---|---|---|---|---|
| Number | Base sequence (5'→3') | Number | Base sequence (5'→3') | Number | Base sequence (5'→3') | Number | Base sequence (5'→3') |
| 1 | AAAA | 21 | GAGA | 41 | TCGA | 61 | GGAC |
| 2 | AAAC | 22 | GGAA | 42 | TCTC | 62 | GGCA |
| 3 | AAAG | 23 | GGAG | 43 | TCTT | 63 | GGCT |
| 4 | AAAT | 24 | GGGA | 44 | TGAA | 64 | GGGG |
| 5 | AACA | 25 | GTCG | 45 | TGAG | 65 | GTAG |
| 6 | AACG | 26 | TAAA | 46 | TGCA | 66 | TCAC |
| 7 | AACT | 27 | TAAC | 47 | TGGA | 67 | TGAC |
| 8 | AAGA | 28 | TAAG | 48 | TGGG | 68 | TGCG |
| 9 | AATT | 29 | TACA | 49 | TGTA | 69 | TGCT |
| 10 | ACAA | 30 | TACC | 50 | TTCT | 70 | TGGC |
| 11 | AGAA | 31 | TACG | 51 | TTTA | 71 | TTAT |
| 12 | AGAG | 32 | TAGA | 52 | TTTC | 72 | GCAA |
| 13 | AGCA | 33 | TAGG | 53 | TTTT | 73 | GCAG |
| 14 | AGCG | 34 | TATT | 54 | AACC | 74 | GTAA |
| 15 | AGCT | 35 | TCAA | 55 | AAGG | 75 | GTCA |
| 16 | AGGA | 36 | TCAG | 56 | ACTT | 76 | TCGG |
| 17 | AGGC | 37 | TCCA | 57 | AGTT | 77 | TCTA |
| 18 | AGGG | 38 | TCCC | 58 | GAAG | 78 | AGAT |
| 19 | AGTA | 39 | TCCG | 59 | GACA | | |
| 20 | GAAA | 40 | TCCT | 60 | GACC | | |

### [3' Adapter]

The 3' adapter according to the present disclosure is a 5'-adenylated single-stranded DNA in which the base sequence of the 5' end excluding the adenylic acid at the 5' end is any one of the base sequences shown in Table A.

In the 3' adapter according to the present disclosure, the base sequence of the 5' end excluding the adenylic acid at the 5' end is any one of the base sequences of Numbers 1 to 78 in Table A above.

In the 3' adapter according to the present disclosure, the base sequence of the 5' end excluding the adenylic acid at the 5' end is, from the viewpoint of improving sequencing efficiency, preferably any one selected from the group consisting of the base sequences of Numbers 1 to 53 shown in Table A above, more preferably any one selected from the group consisting of the base sequences of Numbers 1 to 3, Numbers 5 to 7, Numbers 10 to 16, Number 18, Number 22, Number 24, Numbers 26 to 29, Numbers 31 to 32, Numbers 34 to 38, Numbers 41 to 48, and Numbers 51 to 52 shown in Table A above, further preferably any one selected from the group consisting of the base sequences of Number 1, Number 3, Number 5, Number 11, Number 12, Number 16, Number 18, Number 22, Number 26, Number 29, Number 32, Number 35, Number 37, Number 42, Number 44, Number 47, and Number 51 shown in Table A above, and particularly preferably any one selected from the group consisting of the base sequences of Number 5, Number 11, Number 16, Number 26, Number 29, Number 35, Number 37, and Number 42 shown in Table A above.

Note that in Table A above, the order of Numbers 1 to 78 representing the base sequences does not have any particular meaning.

In one aspect, the effective read rate is 1.0% or more when a 5'-adenylated single-stranded DNA in which the base sequence of the 5' end excluding the adenylic acid at the 5' end is any one selected from the group consisting of the base sequences of Numbers 1 to 78 shown in Table A above is used as a 3' adapter for sequencing.

In one aspect, the effective read rate is 1.5% or more when a 5'-adenylated single-stranded DNA in which the base sequence of the 5' end excluding the adenylic acid at the 5' end is any one selected from the group consisting of the base sequences of Numbers 1 to 53 shown in Table A above is used as a 3' adapter for sequencing.

In one aspect, the effective read rate is 2.0% or more when a 5'-adenylated single-stranded DNA in which the base sequence of the 5' end excluding the adenylic acid at the 5' end is any one selected from the group consisting of the base sequences of Numbers 1 to 3, Numbers 5 to 7, Numbers 10 to 16, Number 18, Number 22, Number 24, Numbers 26 to 29, Numbers 31 to 32, Numbers 34 to 38, Numbers 41 to 48, and Numbers 51 to 52 shown in Table A above is used as a 3' adapter for sequencing.

In one aspect, the effective read rate is 3.0% or more when a 5'-adenylated single-stranded DNA in which the base sequence of the 5' end excluding the adenylic acid at the 5' end is any one selected from the group consisting of the base sequences of Number 1, Number 3, Number 5, Number 11, Number 12, Number 16, Number 18, Number 22, Number 26, Number 29, Number 32, Number 35, Number 37, Number 42, Number 44, Number 47, and Number 51 shown in Table A above is used as a 3' adapter for sequencing.

In one aspect, the effective read rate is 5.0% or more when a 5'-adenylated single-stranded DNA in which the base sequence of the 5' end excluding the adenylic acid at the 5' end is any one selected from the group consisting of the base sequences of Number 5, Number 11, Number 16, Number 26, Number 29, Number 35, Number 37, and Number 42 shown in Table A above is used as a 3' adapter for sequencing.

In the present disclosure, the effective read rate (also referred to as the proportion of effective reads) represents the efficiency of target data acquisition in sequencing. The effective read rate is obtained by the rate (%) of the number of effective reads to the total read count. For example, the effective read rate for microRNAs is the proportion of reads whose sequence is the same or substantially the same as the sequence on the miRBase database among all reads obtained by sequencing.

From the viewpoint of improving sequencing efficiency, in the 3' adapter according to the present disclosure, the first base of the 5' end excluding the adenylic acid at the 5' end is adenine, thymine, or guanine, and is preferably adenine or thymine.

From the viewpoint of improving sequencing efficiency, in the 3' adapter according to the present disclosure, the second base of the 5' end excluding the adenylic acid at the 5' end is preferably adenine, guanine, or cytosine.

In the 3' adapter according to the present disclosure, the base sequence of the 5' end excluding the adenylic acid at the 5' end is preferably any one of the base sequences shown in the following Table B (i.e., Number 5, Number 6, Number 7, Number 10, Number 16, Number 44, Number 59, Number 71, and Number 75 in Table A), from the viewpoint of suitably suppressing the formation of adapter dimers and the lot-to-lot variation of T4 RNA Ligase 1.

**[Table 11]**

| Table B | | |
|---|---|---|
| GACA | GTCA | AACT |
| ACAA | TTAT | AGGA |
| AACA | TGAA | AACG |

Hereinafter, in the present disclosure, a ligation method including:
ligating, to the 3' end of each of a plurality of kinds of nucleic acids having different sequences, a 5'-adenylated single-stranded DNA that is a 3' adapter in which the base sequence of the 5' end excluding the adenylic acid at the 5' end is any one of the base sequences shown in Table B above; and after the ligation of the 5'-adenylated single-stranded DNA, ligating, to the 5' end of each of the plurality of kinds of nucleic acids, a single-stranded nucleic acid that is a 5' adapter, is referred to as Embodiment B. In addition, the "5'-adenylated single-stranded DNA that is a 3' adapter in which the base sequence of the 5' end excluding the adenylic acid at the 5' end is any one of the base sequences shown in Table B" is also simply referred to as "the 3' adapter according to Embodiment B".

In the present disclosure, the descriptions regarding Embodiment B are the same as the descriptions in the embodiments of the present disclosure, including definitions, examples, preferred aspects, and the like, unless otherwise specified, except that the base sequence of the 5' end of the 3' adapter, excluding the adenylic acid at the 5' end, is any one of the base sequences shown in Table B above.

When acquiring large-scale data continuously, such as those of next-generation sequencing, a batch effect, which is an error accompanying differences in laboratories and experimenters, often causes a problem. The batch effect not only is a barrier to discovering new biomarkers, but also makes it difficult to operate the discovered diagnostic measurement techniques for a long period of time. A cause of the batch effect in next-generation sequencing includes lot-to-lot variation of reagents used for library preparation. In particular, it is known that T4 RNA Ligase used for the ligation reaction has lot-to-lot variation. While there are two subtypes of T4 RNA Ligase, T4 RNA Ligase 1 and T4 RNA Ligase 2, and further, their variants and the like are also distributed, T4 RNA Ligase 1 is advantageous from the viewpoint of availability. However, T4 RNA Ligase 1 is susceptible to the batch effect due to the lot-to-lot variation. However, according to Embodiment B, it was found that a ligation method in which adapter dimers are unlikely to be formed and which is unlikely to be affected by the lot-to-lot variation of T4 RNA Ligase 1 can be carried out.

As described above, in the ligation of adapters in library preparation of nucleic acids, when surplus 5' adapters and surplus 3' adapters bind to each other, adapter dimers are generated. In addition, T4 RNA Ligase 1 has lot-to-lot variation, and when large-scale data is continuously acquired by next-generation sequencing or the like, a batch effect, which is an error, is likely to occur. The inventors first found that the lot-to-lot variation of T4 RNA Ligase 1 is more likely to occur due to the ligation of the 3' adapter than the ligation of the 5' adapter.

In addition, focusing on the possibility that the base sequence of the 5' end of the 3' adapter affects the ease of formation of adapter dimers, the inventors investigated the relationship between the sequence of 4 bases from the 5' end of the 3' adapter, excluding the adenylic acid at the 5' end, and the ease of formation of adapter dimers. This is because if the amount of the generated adapter dimers is large, the adapter dimers are also amplified by PCR at the time of NGS library preparation, and sequencing efficiency decreases. As a result, it was found that the sequence of the 4 bases was related to the ease of formation of adapter dimers, and the base sequences of the 5' end of the 3' adapter in which the adapter dimers are unlikely to be formed could be specified. Although the embodiments of Embodiment B are not limited by theory, the 3' adapter having the specified base sequence may be easily ligated to the target nucleic acids, and/or may be less likely to be ligated or hybridized with the 5' adapter.

Subsequently, focusing on the possibility that the base sequence of the 5' end of the 3' adapter excluding the adenylic acid at the 5' end also affects the lot-to-lot variation of T4 RNA Ligase 1, the relationship between the sequence of the 4 bases of the 5' end and the lot-to-lot variation of T4 RNA Ligase 1 was investigated in the 3' adapters in which adapter dimers are unlikely to be formed. As a result, it was found that the sequence of the 4 bases was related to the lot-to-lot variation of T4 RNA Ligase 1, and the base sequences of the 5' end of the 3' adapter capable of reducing the lot-to-lot variation could be specified. Although Embodiment B is not limited by theory, the specified base sequences may have approximately the same affinity with T4 RNA ligase regardless of the lot of the T4 RNA ligase, and the ligation efficiency may tend to be homogeneous.

In the 3' adapter according to Embodiment B, from the viewpoint of suitably suppressing the formation of adapter dimers and the lot-to-lot variation of T4 RNA Ligase 1, the base sequence of the 5' end excluding the adenylic acid at the 5' end is preferably ACAA, AACA, AACT, AGGA, or AACG (i.e., Number 5, Number 6, Number 7, Number 10, or Number 16 in Table A), more preferably ACAA, AACA, AACT, or AACG (i.e., Number 5, Number 6, Number 7, or Number 10 in Table A), and further preferably AACA, AACT, or AACG (i.e., Number 5, Number 6, or Number 7 in Table A).

In one aspect, when adapter ligation to a plurality of nucleic acids, PCR, and sequencing are performed using the 3' adapter according to Embodiment B, the number of effective reads is preferably 1.0% or more, more preferably 1.5% or more, further preferably 2.0% or more, particularly preferably 3.0% or more, and extremely preferably 5.0% or more.

In the 3' adapter according to the present disclosure, the 5th and subsequent bases of the base sequence of the 5' end excluding the adenylic acid at the 5' end may be any base sequence. A modified base may or may not be included in the 5th and subsequent bases of the base sequence of the 5' end excluding the adenylic acid at the 5' end. Examples of the modified base include, but are not limited to, 4-acetylcytidine, dihydrouridine, inosine, and 1-methyladenosine.

The 3' adapter according to the present disclosure preferably has a base length excluding the adenylic acid at the 5' end of from 6 to 1000 bases, more preferably from 6 to 100 bases, and further preferably from 10 to 30 bases, from the viewpoint of easy ligation between the adapter and the target nucleic acids.

The nucleotide at the 3' end of the 3' adapter according to the present disclosure is preferably modified with a compound having a modified structure capable of suppressing the binding of nucleic acids.

Examples of the compound having a modified structure capable of suppressing the binding of nucleic acids include a dideoxyribonucleoside triphosphate (ddNTP) and a compound having an amino group. Examples of the dideoxyribonucleoside triphosphate include dideoxycytidine triphosphate (ddCTP), dideoxyguanosine triphosphate (ddGTP), dideoxythymidine triphosphate (ddTTP), and dideoxyadenosine triphosphate (ddATP). As the compound having a modified structure capable of suppressing the binding of nucleic acids, a dideoxyribonucleoside triphosphate is preferable, and dideoxycytidine triphosphate is more preferable.

### [Plurality of Kinds of Nucleic Acids Having Different Sequences]

In the present disclosure, "a plurality of kinds of nucleic acids having different sequences" means that there are a plurality of kinds of nucleic acids having different base sequences. The aspect of the plurality of kinds of nucleic acids having different sequences is not particularly limited.

The nucleic acids are preferably single-stranded from the viewpoint of being easily ligated with the 3' adapter according to the present disclosure. In the present disclosure, the nucleic acids may be, for example, DNA, RNA, an analog thereof, a fusion thereof, or the like. The nucleic acids may be small RNAs (sRNAs). The small RNAs may be any of microRNAs (miRNAs), piRNAs, and tsRNAs. The nucleic acids may be nucleic acids composed only of bases A, T, G, C, and U, or may not be nucleic acids composed only of bases A, T, G, C, and U. Specifically, examples of the nucleic acids not composed only of bases A, T, G, C, and U include nucleic acids that have undergone a modification such as methylation of DNA or RNA, or an editing such as A-to-I RNA editing.

The origin of the nucleic acids is also not particularly limited, and may be naturally derived nucleic acids (e.g., total RNA) or synthesized RNAs. The origin of the nucleic acids may be, for example, a biological sample, a viral sample, an environmental sample, or an artificial sample.

Examples of the biological sample include a sample derived from an animal, a plant, a fungus, and a bacterium. Examples of the animal include a human and a non-human animal. Examples of the non-human animal include a non-human mammal (e.g., a monkey, a dog, a cat, a mouse, a rat, a rabbit, a cow, a horse, a pig, or a sheep) and a bird (e.g., a chicken or a quail).

More specifically, examples of the sample derived from an animal include serum, plasma, whole blood, urine, feces, saliva, bone marrow fluid, lymph fluid, and a tissue.

Examples of the environmental sample include a sample derived from soil or water.

Examples of the artificial sample include artificial serum, artificial plasma, and artificial urine.

The nucleic acids may be 1 to 300,000 kinds of nucleic acids, 1 to 10,000 kinds of nucleic acids, or 1 to 2,000 kinds of nucleic acids.

The base lengths of the nucleic acids are not particularly limited, and are preferably from 1 to 100 bases in length, more preferably from 10 to 50 bases in length, and further preferably from 16 to 40 bases in length.

### [Method of Ligation]

Regarding the ligation of the 3' adapter according to the present disclosure to the 3' end of each of the plurality of kinds of nucleic acids in the ligation method according to the present disclosure, detailed conditions thereof can be in accordance with a conventional method. For example, the concentration of each component, temperature, time, and the like when ligating the 3' adapter according to the present disclosure to the nucleic acids are not particularly limited.

The reaction temperature when ligating the 3' adapter according to the present disclosure to the nucleic acids is preferably from 10°C to 40°C, more preferably from 15°C to 30°C, further preferably from 17°C to 25°C, and particularly preferably 20°C. The reaction time is preferably from 10 minutes to 2 hours, more preferably from 30 minutes to 90 minutes, further preferably from 45 minutes to 75 minutes, and particularly preferably 1 hour.

One kind of the 3' adapter according to the present disclosure may be ligated to the 3' ends of the plurality of kinds of nucleic acids, or two or more kinds of the 3' adapters according to the present disclosure may be ligated thereto.

### (Ligase)

In the ligation method according to the present disclosure, an enzyme for ligation (also referred to as a ligase) in ligating the 3' adapter according to the present disclosure to the 3' end of each of the plurality of kinds of nucleic acids is not particularly limited, and a known enzyme can be used. Only one kind of ligase may be used, or multiple kinds of ligases may be used.

The ligase used for the ligation of the 3' adapter is preferably at least one selected from the group consisting of T4 RNA Ligase 1, T4 RNA Ligase 2, T4 DNA Ligase, and variants thereof. From the viewpoint of improving sequencing efficiency, T4 RNA Ligase 2 or a variant thereof is more preferable.

In Embodiment B, the ligase used for the ligation of the 3' adapter is preferably at least one selected from the group consisting of T4 RNA Ligase 1, T4 RNA Ligase 2, T4 DNA Ligase, and variants thereof, and from the viewpoint of suitably suppressing the formation of adapter dimers and the lot-to-lot variation of T4 RNA Ligase 1, T4 RNA Ligase 1 is more preferable.

The variant of T4 RNA Ligase 1, the variant of T4 RNA Ligase 2, or the variant of T4 DNA Ligase refers to one having a certain sequence identity, for example, 50% or more, 60% or more, 70% or more, 80% or more, 85% or more, 90% or more, 95% or more, 98% or more, or 99% or more sequence identity to the amino acid sequence of the wild-type T4 RNA Ligase 1, T4 RNA Ligase 2, or T4 DNA Ligase from which it is derived, and having ligation activity between a nucleic acid and an adapter similarly to the wild-type T4 RNA Ligase 1, T4 RNA Ligase 2, or T4 DNA Ligase. The sequence identity here is the sequence identity when the full lengths of the respective ligases are compared.

Here, examples of the method for calculating the identity of the amino acid sequences include known means. For example, the identity can be calculated using a commercially available analysis tool or an analysis tool available through a telecommunication line (the Internet). As an example, the identity of the amino acid sequences can be calculated by using default (initial setting) parameters in the homology algorithm BLAST (Basic local alignment search tool), http://www.ncbi.nlm.nih.gov/BLAST/, of the National Center for Biotechnology Information (NCBI).

In addition, the variant of T4 RNA Ligase 1, the variant of T4 RNA Ligase 2, or the variant of T4 DNA Ligase may be a protein consisting of an amino acid sequence in which one or more amino acids are substituted, deleted, inserted, and/or added in the amino acid sequence of the wild-type T4 RNA Ligase 1, T4 RNA Ligase 2, or T4 DNA Ligase from which it is derived, and having ligation activity between a nucleic acid and an adapter similarly to the wild-type T4 RNA Ligase 1, T4 RNA Ligase 2, or T4 DNA Ligase. Here, "one or more" may be, for example, from 1 to 100, from 1 to 80, more preferably from 1 to 40, further preferably from 1 to 10, particularly preferably from 1 to 5, and extremely preferably from 1 to 3, but is not particularly limited.

Examples of the variant of T4 RNA Ligase 2 include a variant containing an amino acid sequence having 85% or more, 90% or more, 95% or more, 98% or more, or 99% or more identity to the amino acid sequence of positions 1 to 249 from the N-terminus of the wild-type T4 RNA Ligase 2. In addition, examples of the variant of T4 RNA Ligase 2 include a variant containing the amino acid sequence of positions 1 to 249 from the N-terminus of the wild-type T4 RNA Ligase 2, or a variant containing an amino acid sequence in which 1 to 10, 1 to 5, or 1 to 3 amino acids are substituted, deleted, inserted, and/or added in the amino acid sequence of positions 1 to 249 from the N-terminus. Examples of the variant of T4 RNA Ligase 2 include, for example, T4 RNA Ligase 2 truncated, T4 RNA Ligase 2 truncated KQ, and T4 RNA Ligase 2 truncated K227Q, and T4 RNA Ligase 2 truncated or T4 RNA Ligase 2 truncated KQ is preferable.

T4 RNA Ligase 2 truncated is a variant in which the C-terminus of the amino acid sequence of T4 RNA Ligase 2 is deleted, and generally has the amino acid sequence of positions 1 to 249 from the N-terminus of T4 RNA Ligase 2.

T4 RNA Ligase 2 truncated K227Q is a variant in which K (lysine) at position 227 of the amino acid sequence of T4 RNA Ligase 2 truncated is mutated to Q (glutamine).

T4 RNA Ligase 2 truncated KQ is a variant (double point mutant) in which R (arginine) at position 55 is further mutated to K (lysine) with respect to the amino acid sequence of T4 RNA Ligase 2 truncated K227Q.

Examples of the variant of T4 DNA Ligase include, for example, Salt-T4 DNA Ligase.

### <Ligation of 5' Adapter>

The ligation method according to the present disclosure includes, after the ligation of the 3' adapter, ligating, to the 5' end of each of the plurality of kinds of nucleic acids, a single-stranded nucleic acid that is a 5' adapter.

### [5' Adapter]

The 5' adapter is a single-stranded nucleic acid. As long as the 5' adapter is a single-stranded nucleic acid, other characteristics are not particularly limited. The base length of the single-stranded nucleic acid that is a 5' adapter is also not particularly limited, and is preferably from 5 to 100 bases in length, more preferably from 15 to 50 bases in length, and further preferably from 20 to 40 bases in length.

The nucleic acid in the single-stranded nucleic acid that is a 5' adapter may be DNA, RNA, an analog thereof, a fusion thereof, or the like. That is, the 5' adapter may be a single-stranded DNA or a single-stranded RNA. The nucleic acid in the single-stranded nucleic acid may be a nucleic acid composed only of bases A, T, G, C, and U, or may not be a nucleic acid composed only of bases A, T, G, C, and U. Specifically, examples of the nucleic acid not composed only of bases A, T, G, C, and U include a nucleic acid that has undergone a modification such as methylation of DNA or RNA, or an editing such as A-to-I RNA editing.

It is believed that, by the ligation method according to the present disclosure, sequencing efficiency can be improved in a simpler manner regardless of the base sequence of the 5' adapter. Therefore, in the present disclosure, the base sequence of the 5' adapter is not particularly limited.

It is believed that, by the ligation method according to Embodiment B, the formation of adapter dimers and the lot-to-lot variation of T4 RNA Ligase 1 can be suppressed regardless of the base sequence of the 5' adapter. Therefore, also in Embodiment B, the base sequence of the 5' adapter is not particularly limited.

The base sequence of the 5' adapter may be a base sequence having a sequence identity of 80% or more to each of the base sequences represented by SEQ ID NOs: 1 to 6 below. The sequence identity may be 85% or more, 90% or more, 95% or more, or 100% (i.e., the base sequences are completely identical).
(SEQ ID NO: 1) 5'- GUUCAGAGUUCUACAGUCCGACGAUCGGAG -3'
(SEQ ID NO: 2) 5'- GUUCAGAGUUCUACAGUCCGACGAUCCACG -3'
(SEQ ID NO: 3) 5'- GUUCAGAGUUCUACAGUCCGACGAUCUUGU -3'
(SEQ ID NO: 4) 5'- GUUCAGAGUUCUACAGUCCGACGAUC -3'
(SEQ ID NO: 5) 5'- GUUCAGAGUUCUACAGUCCGACGAUCUCAU -3'
(SEQ ID NO: 6) 5'- GUUCAGAGUUCUACAGUCCGACGAUCUGCA -3'

Here, examples of the method for calculating the identity of the base sequences include known means. For example, the identity can be calculated using a commercially available analysis tool or an analysis tool available through a telecommunication line (the Internet). As an example, the identity of the base sequences can be calculated by using default (initial setting) parameters in the homology algorithm BLAST (Basic local alignment search tool), http://www.ncbi.nlm.nih.gov/BLAST/, of the National Center for Biotechnology Information (NCBI).

The base sequence of the 5' adapter may be a base sequence consisting of a base sequence in which one or more bases are substituted, deleted, inserted, and/or added in any of the base sequences represented by SEQ ID NOs: 1 to 6 above, and being capable of ligating to a nucleic acid similarly to the 5' adapter of any of the base sequences represented by SEQ ID NOs: 1 to 6 above. Here, "one or more" may be, for example, from 1 to 80, preferably from 1 to 40, more preferably from 1 to 10, further preferably from 1 to 5, still more preferably from 1 to 3, and particularly preferably 1 or 2, but is not particularly limited.

### [Plurality of Kinds of Nucleic Acids Having Different Sequences]

The plurality of kinds of nucleic acids to which the 5' adapter is ligated are nucleic acids in which the 3' adapter is connected to the respective 3' ends of the plurality of kinds of nucleic acids that are target nucleic acids. The plurality of kinds of nucleic acids to which the 5' adapter is ligated may be those in which a primer or the like is further added. For example, when the plurality of kinds of nucleic acids that are target nucleic acids are RNAs, the plurality of kinds of nucleic acids to which the 5' adapter is ligated may be those in which a reverse transcription primer is annealed to the plurality of kinds of nucleic acids to which the 3' adapter is ligated.

The plurality of kinds of nucleic acids obtained after the ligation of the 3' adapter may be subjected to a purification treatment after the aforementioned operation of ligating the 3' adapter. The purification treatment may be performed by a purification method using an organic solvent, or may be performed by a purification method using a solid phase (e.g., a silica membrane, an ion exchange column, or magnetic beads). Since the formation of adapter dimers can be suppressed by the ligation method according to the present disclosure, the purification treatment does not need to be performed.

### [Method of Ligation]

Regarding the ligation of the 5' adapter to the 5' end of each of the plurality of kinds of nucleic acids in the ligation method according to the present disclosure, detailed conditions thereof can be in accordance with a conventional method. For example, the concentration of each component, temperature, time, and the like when ligating the 5' adapter to the nucleic acids are not particularly limited.

The reaction temperature when ligating the 5' adapter to the nucleic acids is preferably from 10°C to 40°C, more preferably from 15°C to 30°C, further preferably from 17°C to 25°C, and particularly preferably 20°C. The reaction time is preferably from 10 minutes to 2 hours, more preferably from 30 minutes to 90 minutes, further preferably from 45 minutes to 75 minutes, and particularly preferably 1 hour.

One kind of 5' adapter may be ligated to the 5' ends of the plurality of kinds of nucleic acids, or two or more kinds of 5' adapters may be ligated thereto.

### (Ligase)

In the ligation method according to the present disclosure, an enzyme for ligation (also referred to as a ligase) in ligating the 5' adapter to the 5' end of each of the plurality of kinds of nucleic acids is not particularly limited, and a known enzyme can be used. Only one kind of ligase may be used, or a plurality of kinds of ligases may be used.

The ligase used for the ligation of the 5' adapter is preferably at least one selected from the group consisting of T4 RNA Ligase 1, T4 RNA Ligase 2, T4 DNA Ligase, and variants thereof, and T4 RNA Ligase 1 or a variant thereof is more preferable.

The descriptions regarding definitions, examples, preferred aspects, and the like of the variant of T4 RNA Ligase 1, the variant of T4 RNA Ligase 2, and the variant of T4 DNA Ligase are the same as the descriptions regarding definitions, examples, preferred aspects, and the like of the variant of T4 RNA Ligase 1, the variant of T4 RNA Ligase 2, and the variant of T4 DNA Ligase described in the foregoing <Ligation of 3' Adapter>.

The kind of ligase used in the above-described ligation of the 3' adapter and ligation of the 5' adapter may be the same or different. In Embodiment B, from the viewpoint of simplicity, the ligation of the 3' adapter and the ligation of the 5' adapter are preferably performed using the same ligase, and from the viewpoint that the ligation method according to Embodiment B is particularly useful, it is more preferable to perform both using T4 RNA Ligase 1.

### <<Method of Suppressing Adapter Dimer Formation>>

The method of suppressing adapter dimer formation according to the present disclosure includes:
ligating, to the 3' end of each of a plurality of kinds of nucleic acids having different sequences, a 5'-adenylated single-stranded DNA that is a 3' adapter in which the base sequence of the 5' end, excluding the adenylic acid at the 5' end, is any one of base sequences shown in Table A above; and
after the ligating of the 5'-adenylated single-stranded DNA, ligating, to the 5' end of each of the plurality of kinds of nucleic acids, a single-stranded nucleic acid that is a 5' adapter.

In the method of suppressing adapter dimer formation according to the present disclosure, descriptions of definitions, examples, preferred aspects, and the like regarding the ligation of the 3' adapter and the ligation of the 5' adapter are the same as the descriptions of definitions, examples, preferred aspects, and the like described in the foregoing <Ligation of 3' Adapter> and <Ligation of 5' Adapter>, respectively.

In the method of suppressing adapter dimer formation according to the present disclosure, as described above, the base sequence of the 5' end of the 3' adapter according to the present disclosure is preferably any one selected from the group consisting of the base sequences of Numbers 1 to 53 shown in Table A.

In the method of suppressing adapter dimer formation according to the present disclosure, as described above, the ligase used for the ligation of the 3' adapter and the ligase used for the ligation of the 5' adapter are each independently preferably at least one selected from the group consisting of T4 RNA Ligase 1, T4 RNA Ligase 2, T4 DNA ligase, and a variant thereof.

### <<Method of Preparing NGS Library>>

The method of preparing an NGS library according to the present disclosure includes:
ligating, to a plurality of kinds of nucleic acids having different sequences, a 5'-adenylated single-stranded DNA that is the 3' adapter according to the present disclosure and a single-stranded nucleic acid that is a 5' adapter by the ligation method according to the present disclosure;
synthesizing a cDNA by a reverse transcription reaction from the plurality of kinds of nucleic acids to which the 3' adapter and the 5' adapter have been ligated; and
amplifying the obtained cDNA by PCR.

The method of preparing an NGS library according to Embodiment B includes:
ligating, to a plurality of kinds of RNAs having different sequences, a 5'-adenylated single-stranded DNA that is the 3' adapter according to Embodiment B and a single-stranded nucleic acid that is a 5' adapter by the ligation method according to Embodiment B;
synthesizing a cDNA by a reverse transcription reaction from the plurality of kinds of RNAs to which the 3' adapter and the 5' adapter have been ligated; and amplifying the obtained cDNA by PCR.

### <Ligation>

In the method of preparing an NGS library according to the present disclosure, the description regarding ligating to a plurality of kinds of nucleic acids a 5'-adenylated single-stranded DNA that is the 3' adapter according to the present disclosure and a single-stranded nucleic acid that is a 5' adapter by the ligation method according to the present disclosure is as described above.

### <Synthesis of cDNA>

The method of preparing an NGS library according to the present disclosure includes synthesizing a cDNA by a reverse transcription reaction from the plurality of kinds of nucleic acids to which the 3' adapter and the 5' adapter have been ligated.

In one aspect, the reverse transcription reaction includes annealing a reverse transcription primer to the plurality of kinds of nucleic acids to which the 3' adapter and the 5' adapter have been ligated. Alternatively, the reverse transcription primer may be annealed after the ligation of the 3' adapter and before the ligation of the 5' primer. The reverse transcription primer may have a sequence that further adds a unique sequence to each of the plurality of kinds of nucleic acids. Examples of the unique sequence include a UMI (Unique molecular identifier) sequence. By attaching a sequence unique to each of the plurality of kinds of nucleic acids to the cDNA, even when the cDNA is amplified by PCR as described later, the abundance of each nucleic acid that was present before the amplification can be determined.

The method of the reverse transcription reaction for synthesizing the cDNA is not particularly limited, and for example, a known reverse transcriptase and conditions for the reverse transcription reaction and the like can be used. For example, the reverse transcription reaction can be performed by reverse-transcribing the plurality of kinds of nucleic acids to which the 3' adapter according to the present disclosure and the 5' adapter have been ligated in the presence of a reverse transcriptase, a primer, a dNTP mix, and an appropriate buffer.

### <Amplification of cDNA>

The method of preparing an NGS library according to the present disclosure includes amplifying the obtained cDNA by PCR. The method of the PCR is not particularly limited, and for example, a known PCR enzyme and PCR conditions and the like can be used.

### <Order of Each Method>

The method of preparing an NGS library according to the present disclosure preferably includes:
ligating, to a plurality of kinds of nucleic acids having different sequences, a 5'-adenylated single-stranded DNA that is the 3' adapter according to the present disclosure and a single-stranded nucleic acid that is a 5' adapter by the ligation method according to the present disclosure;
synthesizing a cDNA by a reverse transcription reaction from the plurality of kinds of nucleic acids to which the 3' adapter and the 5' adapter have been ligated; and
amplifying the obtained cDNA by PCR,
in this order.

When the ligation of the 5' adapter is performed after the ligation of the 3' adapter according to the present disclosure to the plurality of kinds of nucleic acids, "ligating a single-stranded nucleic acid that is a 5' adapter to the 5' end of each of the plurality of kinds of nucleic acids" means ligating a single-stranded nucleic acid that is a 5' adapter to the 5' end of each of the plurality of kinds of nucleic acids to which the 3' adapter according to the present disclosure has been ligated.

On the other hand, when the ligation of the 3' adapter according to the present disclosure is performed after the ligation of the 5' adapter to the plurality of kinds of nucleic acids, "ligating a 5'-adenylated single-stranded DNA that is a 3' adapter to the 3' end of each of the plurality of kinds of nucleic acids" means ligating a 5'-adenylated single-stranded DNA that is the 3' adapter according to the present disclosure to the 3' end of each of the plurality of kinds of nucleic acids to which the 5' adapter has been ligated.

In the present disclosure, the descriptions regarding the method of preparing an NGS library according to Embodiment B are the same as the above descriptions regarding the method of preparing an NGS library according to the present disclosure, except that the ligation method according to Embodiment B is used and the plurality of kinds of nucleic acids are RNAs, unless otherwise specified.

### <<NGS Library Preparation Kit>>

The NGS library preparation kit according to the present disclosure includes a 5'-adenylated single-stranded DNA contained in a first container, wherein a base sequence of the 5' end of the 5'-adenylated single-stranded DNA, excluding the adenylic acid at the 5' end, is any one of base sequences shown in the following Table A.

**[Table 12]**

| Table A | | | | | | | |
|---|---|---|---|---|---|---|---|
| Number | Base sequence (5'→3') | Number | Base sequence (5'→3') | Number | Base sequence (5'→3') | Number | Base sequence (5'→3') |
| 1 | AAAA | 21 | GAGA | 41 | TCGA | 61 | GGAC |
| 2 | AAAC | 22 | GGAA | 42 | TCTC | 62 | GGCA |
| 3 | AAAG | 23 | GGAG | 43 | TCTT | 63 | GGCT |
| 4 | AAAT | 24 | GGGA | 44 | TGAA | 64 | GGGG |
| 5 | AACA | 25 | GTCG | 45 | TGAG | 65 | GTAG |
| 6 | AACG | 26 | TAAA | 46 | TGCA | 66 | TCAC |
| 7 | AACT | 27 | TAAC | 47 | TGGA | 67 | TGAC |
| 8 | AAGA | 28 | TAAG | 48 | TGGG | 68 | TGCG |
| 9 | AATT | 29 | TACA | 49 | TGTA | 69 | TGCT |
| 10 | ACAA | 30 | TACC | 50 | TTCT | 70 | TGGC |
| 11 | AGAA | 31 | TACG | 51 | TTTA | 71 | TTAT |
| 12 | AGAG | 32 | TAGA | 52 | TTTC | 72 | GCAA |
| 13 | AGCA | 33 | TAGG | 53 | TTTT | 73 | GCAG |
| 14 | AGCG | 34 | TATT | 54 | AACC | 74 | GTAA |
| 15 | AGCT | 35 | TCAA | 55 | AAGG | 75 | GTCA |
| 16 | AGGA | 36 | TCAG | 56 | ACTT | 76 | TCGG |
| 17 | AGGC | 37 | TCCA | 57 | AGTT | 77 | TCTA |
| 18 | AGGG | 38 | TCCC | 58 | GAAG | 78 | AGAT |
| 19 | AGTA | 39 | TCCG | 59 | GACA | | |
| 20 | GAAA | 40 | TCCT | 60 | GACC | | |

In the NGS library preparation kit according to the present disclosure, the base sequence of the 5' end of the 5'-adenylated single-stranded DNA is preferably any one selected from the group consisting of the base sequences of Numbers 1 to 53 shown in Table A, from the viewpoint of improving sequencing efficiency.

The NGS library preparation kit according to Embodiment B includes a 5'-adenylated single-stranded DNA contained in a first container, wherein the base sequence of the 5' end of the 5'-adenylated single-stranded DNA, excluding the adenylic acid at the 5' end, is any one of the base sequences shown in the following Table B.

**[Table 13]**

| Table B | | |
|---|---|---|
| GACA | GTCA | AACT |
| ACAA | TTAT | AGGA |
| AACA | TGAA | AACG |

In the NGS library preparation kit according to Embodiment B, from the viewpoint of suitably suppressing the formation of adapter dimers and the lot-to-lot variation of T4 RNA Ligase 1, the base sequence of the 5' end of the 5'-adenylated single-stranded DNA is preferably ACAA, AACA, AACT, AGGA, or AACG (i.e., Number 5, Number 6, Number 7, Number 10, or Number 16 in Table A), more preferably ACAA, AACA, AACT, or AACG (i.e., Number 5, Number 6, Number 7, or Number 10 in Table A), and further preferably AACA, AACT, or AACG (i.e., Number 5, Number 6, or Number 7 in Table A).

The descriptions of the 3' adapter in the NGS library preparation kit according to the present disclosure are the same as the descriptions of the "3' adapter" described in the section of <<Ligation Method>>, including definitions, examples, preferred aspects, and the like.

The container may contain a buffer for stably preserving the 3' adapter according to the present disclosure. Examples of components contained in the buffer include Tris-hydrochloric acid (Tris-HCl), Triton (registered trademark) X, potassium chloride, sodium chloride, dithiothreitol (DTT), ethylenediaminetetraacetic acid (EDTA), bovine serum albumin (BSA), and glycerol.

### <Other Components>

The kit according to the present disclosure may further include at least one selected from the group consisting of a 5' adapter, a ligase, a reverse transcriptase, a reverse transcription primer, a PCR enzyme, and a PCR primer, in a manner in which each of these is contained in an individual container. From the viewpoint of being able to suppress the lot-to-lot variation of T4 RNA Ligase 1, the kit according to Embodiment B may further include T4 RNA Ligase 1 contained in a second container. As the ligase, T4 RNA Ligase 1 and a ligase different from T4 RNA Ligase 1 may be included in a manner in which each of these is contained in an individual container.

In addition, the kit according to the present disclosure may include an instruction manual.

The descriptions regarding the 5' adapter and the ligase are the same as the respective descriptions of the "5' adapter" and the "ligase" described in the section of <<Ligation Method>>, including definitions, examples, preferred aspects, and the like.

The reverse transcriptase, the reverse transcription primer, the PCR enzyme, and the PCR primer may be known ones.

### <<3' Adapter for Preparing Nucleic Acid Library>>

The 3' adapter for preparing a nucleic acid library according to the present disclosure is a single-stranded DNA in which a nucleotide at the 5' end is adenylated, wherein the base sequence of the 5' end of the single-stranded DNA, excluding the adenylic acid at the 5' end, is any one of the base sequences shown in the following Table A.

**[Table 14]**

| Table A | | | | | | | |
|---|---|---|---|---|---|---|---|
| Number | Base sequence (5'→3') | Number | Base sequence (5'→3') | Number | Base sequence (5'→3') | Number | Base sequence (5'→3') |
| 1 | AAAA | 21 | GAGA | 41 | TCGA | 61 | GGAC |
| 2 | AAAC | 22 | GGAA | 42 | TCTC | 62 | GGCA |
| 3 | AAAG | 23 | GGAG | 43 | TCTT | 63 | GGCT |
| 4 | AAAT | 24 | GGGA | 44 | TGAA | 64 | GGGG |
| 5 | AACA | 25 | GTCG | 45 | TGAG | 65 | GTAG |
| 6 | AACG | 26 | TAAA | 46 | TGCA | 66 | TCAC |
| 7 | AACT | 27 | TAAC | 47 | TGGA | 67 | TGAC |
| 8 | AAGA | 28 | TAAG | 48 | TGGG | 68 | TGCG |
| 9 | AATT | 29 | TACA | 49 | TGTA | 69 | TGCT |
| 10 | ACAA | 30 | TACC | 50 | TTCT | 70 | TGGC |
| 11 | AGAA | 31 | TACG | 51 | TTTA | 71 | TTAT |
| 12 | AGAG | 32 | TAGA | 52 | TTTC | 72 | GCAA |
| 13 | AGCA | 33 | TAGG | 53 | TTTT | 73 | GCAG |
| 14 | AGCG | 34 | TATT | 54 | AACC | 74 | GTAA |
| 15 | AGCT | 35 | TCAA | 55 | AAGG | 75 | GTCA |
| 16 | AGGA | 36 | TCAG | 56 | ACTT | 76 | TCGG |
| 17 | AGGC | 37 | TCCA | 57 | AGTT | 77 | TCTA |
| 18 | AGGG | 38 | TCCC | 58 | GAAG | 78 | AGAT |
| 19 | AGTA | 39 | TCCG | 59 | GACA | | |
| 20 | GAAA | 40 | TCCT | 60 | GACC | | |

In the 3' adapter for preparing a nucleic acid library according to the present disclosure, the base sequence of the 5' end excluding the adenylic acid at the 5' end is a base sequence of any of Numbers 1 to 78 in Table A above.

In the 3' adapter according to the present disclosure, from the viewpoint of improving sequencing efficiency, the base sequence of the 5' end excluding the adenylic acid at the 5' end is preferably any one selected from the group consisting of the base sequences of Numbers 1 to 53 shown in Table A above, more preferably any one selected from the group consisting of the base sequences of Numbers 1 to 3, Numbers 5 to 7, Numbers 10 to 16, Number 18, Number 22, Number 24, Numbers 26 to 29, Numbers 31 to 32, Numbers 34 to 38, Numbers 41 to 48, and Numbers 51 to 52 shown in Table A above, further preferably any one selected from the group consisting of the base sequences of Number 1, Number 3, Number 5, Number 11, Number 12, Number 16, Number 18, Number 22, Number 26, Number 29, Number 32, Number 35, Number 37, Number 42, Number 44, Number 47, and Number 51 shown in Table A above, and still more preferably any one selected from the group consisting of the base sequences of Number 5, Number 11, Number 16, Number 26, Number 29, Number 35, Number 37, and Number 42 shown in Table A above.

The 3' adapter for preparing a nucleic acid library according to Embodiment B is a single-stranded DNA in which a nucleotide at the 5' end is adenylated, wherein the base sequence of the 5' end of the single-stranded DNA, excluding the adenylic acid at the 5' end, is any one of the base sequences shown in the following Table B.

**[Table 15]**

| Table B | | |
|---|---|---|
| GACA | GTCA | AACT |
| ACAA | TTAT | AGGA |
| AACA | TGAA | AACG |

In the 3' adapter for preparing a nucleic acid library according to Embodiment B, from the viewpoint of suitably suppressing the formation of adapter dimers and the lot-to-lot variation of T4 RNA Ligase 1, the base sequence of the 5' end excluding the adenylic acid at the 5' end is preferably ACAA, AACA, AACT, AGGA, or AACG (i.e., Number 5, Number 6, Number 7, Number 10, or Number 16 in Table A), more preferably ACAA, AACA, AACT, or AACG (i.e., Number 5, Number 6, Number 7, or Number 10 in Table A), and further preferably AACA, AACT, or AACG (i.e., Number 5, Number 6, or Number 7 in Table A).

In the 3' adapter for preparing a nucleic acid library according to Embodiment B, the details of the 3' adapter are as in the 3' adapter according to Embodiment B described above.

### <<3' Adapter for Ligation>>

The 3' adapter for ligation according to Embodiment B is a single-stranded DNA in which a nucleotide at the 5' end is adenylated, wherein the base sequence of the 5' end of the single-stranded DNA, excluding the adenylic acid at the 5' end, is any one of the base sequences shown in the following Table B.

**[Table 16]**

| Table B | | |
|---|---|---|
| GACA | GTCA | AACT |
| ACAA | TTAT | AGGA |
| AACA | TGAA | AACG |

In the 3' adapter for ligation according to Embodiment B, from the viewpoint of suitably suppressing the formation of adapter dimers and the lot-to-lot variation of T4 RNA Ligase 1, the base sequence of the 5' end excluding the adenylic acid at the 5' end is preferably ACAA, AACA, AACT, AGGA, or AACG (i.e., Number 5, Number 6, Number 7, Number 10, or Number 16 in Table A), more preferably ACAA, AACA, AACT, or AACG (i.e., Number 5, Number 6, Number 7, or Number 10 in Table A), and further preferably AACA, AACT, or AACG (i.e., Number 5, Number 6, or Number 7 in Table A).

In the 3' adapter for ligation according to Embodiment B, the details of the 3' adapter are as in the 3' adapter according to Embodiment B described above.

### Examples

Next, embodiments of the present disclosure will be specifically described by way of Examples. However, embodiments of the present disclosure are not limited to these Examples.

Example 1: Examination of Base Sequences of 3' Adapters for Suppressing Adapter Dimer Formation and Obtaining Sufficient Effective Read Number

In this Example, 3' adapters capable of suppressing adapter dimer formation and obtaining a sufficient number of effective reads were screened. This is because if the amount of the generated adapter dimers is large, the adapter dimers are also amplified by PCR during the library preparation, and sequencing efficiency decreases.

### <Preparation of 3' Adapters>

Since the 3' adapter according to the present disclosure is ligated to a plurality of kinds of nucleic acids at the 5' end side of the 3' adapter, it is believed that the sequence on the 5' end side of the 3' adapter influences the performance of the 3' adapter.

Therefore, 5'-adenylated single-stranded DNAs (hereinafter also referred to as 5'-end randomized DNAs; the base sequences are shown by SEQ ID NO: 7) in which the 1st to 4th bases of the base sequence of the 5' end excluding the adenylic acid at the 5' end were randomized were purchased. The 3' ends of the 5'-end randomized DNAs are blocked by dideoxycytidine modification.

### (SEQ ID NO: 7) 5'-App/NNNNGTAGGCACCATCAAT/3'ddC

In SEQ ID NO: 7, N each independently represents A, T, G, or C. That is, 256 (= 4⁴) kinds of 5'-end randomized DNAs were obtained.

### <Preparation of 5' Adapter>

A 5' adapter is ligated to the plurality of kinds of nucleic acids at the 3' end side. As the 5' adapter, the following 6 kinds of base sequences (SEQ ID NO: 1 to SEQ ID NO: 6) were purchased.
(SEQ ID NO: 1) 5'-GUUCAGAGUUCUACAGUCCGACGAUCGGAG-3'
(SEQ ID NO: 2) 5'-GUUCAGAGUUCUACAGUCCGACGAUCCACG-3'
(SEQ ID NO: 3) 5'-GUUCAGAGUUCUACAGUCCGACGAUCUUGU-3'
(SEQ ID NO: 4) 5'-GUUCAGAGUUCUACAGUCCGACGAUC-3'
(SEQ ID NO: 5) 5'-GUUCAGAGUUCUACAGUCCGACGAUCUCAU-3'
(SEQ ID NO: 6) 5'-GUUCAGAGUUCUACAGUCCGACGAUCUGCA-3'

### <Preparation of NGS Library>

Using the 3' adapters and the 5' adapter respectively, an NGS library of small RNAs (in this Example, human-derived serum RNA was used) was prepared. The NGS library was prepared using a method with reference to Seda Eminaga et al., Quantification of microRNA Expression with Next-Generation Sequencing, Curr Protoc Mol Biol. 2013 July; 04:Unit 4.17. That is, operations of [1] ligation of the 3' adapters, [2] annealing of a reverse transcription primer, [3] ligation of the 5' adapter, [4] reverse transcription, [5] PCR, and [6] purification of the PCR product were performed.
[1] Ligation of 3' adapters
   Nuclease free water 1.0 µL
   T4 RNA Ligase Reaction Buffer (NEB/M0242) 1.0 µL
   100% DMSO (13445-74/Nacalai Tesque) 1.0 µL
   50% PEG (NEB/B1004) 1.0 µL
   Total RNA (human serum-derived RNA) 4.0 µL
   To a mix of the foregoing, 10 µM 3' adapters of SEQ ID NO: 7 were added, and the mixture was incubated at 90°C for 30 seconds.
   T4 RNA Ligase 2, truncated (NEB/M0242) 1.5 µL
   SUPERase inhibitor (Thermo Fisher/AM2694) 0.5 µL

Further, the above-described mix was added, and the total RNA and the 3' adapters were ligated by incubation at 22°C for 60 minutes.

### [2] Annealing of an RT primer

To the solution obtained by the operation of [1], 1 µL of a 10 µM RT primer was added, and the mixture was incubated at 90°C for 30 seconds, and further incubated at 65°C for 5 minutes.

The base sequence of the RT primer is shown in SEQ ID NO: 8 below. The portion represented by N is a UMI sequence. In the following sequence, N each independently represents A, T, G, or C.
(SEQ ID NO: 8) 5'-GTGACTGGAGTTCAGACGTGTGCTCTTCCGATCTNNNNNNNNNNNNATTGATGGT GCCTAC-3'

### [3] Ligation of a 5' adapter

Nuclease free water 0.8 µL
ATP (NEB/0204M) 1.4 µL
T4 RNA Ligase 1 (NEB/0204M) 1.5 µL

To the solution obtained by the operation of [2], the above-described mix and a 10 µM 5' adapter, which has each of the base sequences of SEQ ID NO: 1 to SEQ ID NO: 6, were added, and the mixture was incubated at 20°C for 60 minutes.

### [4] Reverse transcription

Nuclease free water 3.5 µL
25 mM dNTP mix (Nippon Gene/312-07271) 0.3 µL
5×FS Buffer (Thermo Fisher/18080093) 1.5 µL
0.1M DTT (Thermo Fisher/18080093) 1.5 µL
RNase OUT (Thermo Fisher/10777019) 0.375 µL
SuperScript III (Thermo Fisher/18080093) 1.5 µL

Further, to 7.5 µL of the solution obtained by the above-described incubation, the above-described mix was added, and the mixture was incubated at 48°C for 30 seconds and further incubated at 85°C for 5 minutes.

The base sequence of the RT primer is as shown in SEQ ID NO: 8 above.

### [5] PCR

Nuclease free water 24.1 µL
Phusion HF Buffer (NEB/M0530L) 10 µL
25 mM dNTP mix (Nippon Gene/312-07271) 0.4 µL
Phusion High-Fidelity DNA Polymerase (NEB/M0530L) 0.5 µL
10 µM PCR primer R 2.5 µL
10 µM PCR primer F 2.5 µL

To 10 µL of the solution obtained by the operation of [4], the above-described mix was added, and the mixture was incubated at 98°C for 30 seconds. Thereafter, PCR performing 18 cycles of a cycle of 98°C for 10 seconds, 60°C for 20 seconds, and 72°C for 20 seconds was performed.

The base sequence of the PCR primer R is shown in SEQ ID NO: 9 below.
(SEQ ID NO: 9) 5'-CAAGCAGAAGACGGCATACGAGATCGTGATGCTCCACCGATAAATATTAGCCCG T-3'

The base sequence of the PCR primer F is shown in SEQ ID NO: 10 below.
(SEQ ID NO: 10) 5'-AATGATACGGCGACCACCGAGATCTACACGTTCAGAGTTCTACAGTCCGA-3'

The ligation was respectively performed using T4 RNA ligase 2 truncated (New England Biolabs Inc.) in the ligation of the 3' adapters and T4 RNA ligase 1 (New England Biolabs Inc.) in the ligation of the 5' adapter, as the ligase.

### <Sequencing>

The completed library was sequenced with Illumina Nextseq 550, and the effective read rates were determined.

### <Results>

### (Effective Read Rate)

The effective read rates with 256 kinds of 3' adapters for each of the 6 kinds of 5' adapters are shown in Tables 9 to 14. The base sequences shown in Tables 9 to 14 are only the 4 bases of the 5' end excluding the adenylic acid at the 5' end, that is, only the base sequence corresponding to NNNN of SEQ ID NO: 7.

In Tables 9 to 14, the base sequences accompanied by numbers (Number 1 to Number 78) are working examples according to the present disclosure, which had an effective read rate of 1.0% or more in common for all 6 kinds of 5' adapters. On the other hand, the base sequences without numbers are comparative examples in the present disclosure. The number for each base sequence corresponds to the number for the base sequence described in Table A in the present disclosure.

**[Table 17]**

| Number | Base sequence (5'→3') | 5' adapter (SEQ ID NO: 1) | 5' adapter (SEQ ID NO: 2) | 5' adapter (SEQ ID NO: 3) | 5' adapter (SEQ ID NO: 4) | 5' adapter (SEQ ID NO: 5) | 5' adapter (SEQ ID NO: 6) |
|---|---|---|---|---|---|---|---|
| 1 | AAAA | 7.26 | 27.89 | 30.13 | 13.17 | 7.76 | 4.85 |
| 2 | AAAC | 2.48 | 10.23 | 10.45 | 4.45 | 3.28 | 4.74 |
| 3 | AAAG | 3.60 | 11.18 | 17.89 | 9.21 | 11.65 | 5.13 |
| 4 | AAAT | 1.75 | 10.15 | 6.02 | 3.85 | 2.17 | 2.32 |
| 5 | AACA | 5.35 | 20.69 | 23.70 | 13.92 | 9.67 | 7.98 |
| 54 | AACC | 1.47 | 5.95 | 6.00 | 5.09 | 2.15 | 5.06 |
| 6 | AACG | 3.09 | 7.23 | 9.06 | 3.75 | 4.85 | 4.45 |
| 7 | AACT | 2.76 | 14.47 | 11.62 | 7.17 | 3.59 | 13.75 |
| 8 | AAGA | 1.83 | 8.11 | 13.59 | 5.70 | 6.58 | 4.81 |
| | AAGC | 0.18 | 0.21 | 1.27 | 0.72 | 0.19 | 0.54 |
| 55 | AAGG | 1.20 | 5.04 | 6.10 | 2.32 | 3.57 | 3.09 |
| | AAGT | 0.24 | 2.24 | 1.96 | 1.00 | 1.08 | 0.84 |
| | AATA | 0.98 | 7.89 | 4.12 | 2.58 | 1.74 | 1.15 |
| | AATC | 0.26 | 1.24 | 0.82 | 0.54 | 0.30 | 0.66 |
| | AATG | 0.20 | 2.03 | 0.65 | 0.55 | 0.57 | 0.24 |
| 9 | AATT | 1.67 | 34.97 | 4.16 | 10.80 | 2.24 | 6.10 |
| 10 | ACAA | 2.55 | 19.56 | 13.58 | 10.74 | 5.05 | 5.73 |
| | ACAC | 0.32 | 2.20 | 4.69 | 2.18 | 1.05 | 1.95 |
| | ACAG | 0.90 | 13.37 | 10.20 | 5.30 | 3.23 | 3.10 |
| | ACAT | 0.20 | 6.08 | 3.46 | 1.82 | 0.93 | 0.96 |
| | ACCA | 0.84 | 11.85 | 10.25 | 6.62 | 2.38 | 3.54 |
| | ACCC | 0.36 | 5.13 | 4.27 | 2.67 | 1.54 | 2.42 |
| | ACCG | 0.27 | 4.27 | 2.71 | 1.60 | 1.03 | 1.11 |
| | ACCT | 0.42 | 5.64 | 3.68 | 1.95 | 1.43 | 1.82 |
| | ACGA | 0.59 | 6.77 | 5.19 | 2.78 | 1.21 | 2.13 |
| | ACGC | 0.10 | 0.69 | 1.10 | 0.38 | 0.30 | 0.51 |
| | ACGG | 0.33 | 3.15 | 1.42 | 0.61 | 0.49 | 1.18 |
| | ACGT | 0.17 | 2.31 | 1.17 | 0.42 | 0.32 | 0.60 |
| | ACTA | 0.46 | 6.76 | 5.07 | 2.48 | 0.74 | 1.41 |
| | ACTC | 0.58 | 11.97 | 5.33 | 4.27 | 2.13 | 4.42 |
| | ACTG | 0.32 | 2.41 | 2.65 | 1.38 | 0.96 | 0.48 |
| 56 | ACTT | 1.12 | 19.01 | 9.96 | 5.06 | 3.22 | 3.09 |
| 11 | AGAA | 10.38 | 19.54 | 36.63 | 10.76 | 8.50 | 11.36 |
| | AGAC | 3.24 | 1.82 | 6.88 | 0.76 | 1.53 | 4.92 |
| 12 | AGAG | 3.76 | 3.25 | 16.48 | 6.03 | 6.54 | 4.67 |
| 78 | AGAT | 1.19 | 2.11 | 4.01 | 1.51 | 1.44 | 1.21 |
| 13 | AGCA | 2.90 | 2.57 | 17.62 | 5.62 | 3.33 | 4.65 |
| | AGCC | 0.86 | 0.91 | 3.50 | 1.67 | 0.76 | 1.84 |
| 14 | AGCG | 2.62 | 2.11 | 9.01 | 2.24 | 3.68 | 3.47 |
| 15 | AGCT | 2.09 | 2.81 | 6.40 | 2.62 | 2.36 | 2.05 |
| 16 | AGGA | 5.91 | 12.54 | 18.38 | 6.51 | 5.90 | 5.98 |
| 17 | AGGC | 2.13 | 1.56 | 6.68 | 2.02 | 2.06 | 1.77 |
| 18 | AGGG | 4.44 | 8.27 | 11.63 | 6.28 | 4.88 | 6.50 |

**[Table 18]**

| Number | Base sequence (5'→3') | 5' adapter (SEQ ID NO: 1) | 5' adapter (SEQ ID NO: 2) | 5' adapter (SEQ ID NO: 3) | 5' adapter (SEQ ID NO: 4) | 5' adapter (SEQ ID NO: 5) | 5' adapter (SEQ ID NO: 6) |
|---|---|---|---|---|---|---|---|
| | AGGT | 0.52 | 1.84 | 1.97 | 0.86 | 0.62 | 1.07 |
| 19 | AGTA | 2.58 | 6.57 | 7.38 | 2.93 | 1.57 | 2.08 |
| | AGTC | 0.05 | 0.30 | 0.16 | 0.11 | 0.06 | 0.12 |
| | AGTG | 0.68 | 1.76 | 1.49 | 1.30 | 1.15 | 0.64 |
| 57 | AGTT | 1.40 | 4.37 | 2.51 | 1.79 | 1.67 | 1.63 |
| | ATAA | 0.57 | 3.98 | 1.74 | 1.91 | 1.07 | 1.12 |
| | ATAC | 0.21 | 1.51 | 0.69 | 0.61 | 0.45 | 0.56 |
| | ATAG | 0.26 | 1.52 | 0.79 | 1.06 | 0.72 | 0.61 |
| | ATAT | 0.21 | 7.80 | 1.39 | 1.49 | 0.28 | 0.76 |
| | ATCA | 0.43 | 4.47 | 2.27 | 2.29 | 1.10 | 1.29 |
| | ATCC | 0.13 | 0.97 | 0.49 | 0.46 | 0.12 | 0.51 |
| | ATCG | 0.07 | 0.72 | 0.44 | 0.48 | 0.25 | 0.23 |
| | ATCT | 0.26 | 5.11 | 1.15 | 1.23 | 0.63 | 0.93 |
| | ATGA | 0.19 | 1.53 | 0.63 | 0.88 | 0.56 | 0.47 |
| | ATGC | 0.05 | 0.27 | 0.14 | 0.16 | 0.09 | 0.12 |
| | ATGG | 0.12 | 0.78 | 0.25 | 0.42 | 0.24 | 0.26 |
| | ATGT | 0.04 | 0.53 | 0.17 | 0.24 | 0.13 | 0.17 |
| | ATTA | 0.38 | 7.17 | 1.14 | 1.26 | 0.28 | 1.50 |
| | ATTC | 0.06 | 4.63 | 1.46 | 1.79 | 0.63 | 1.35 |
| | ATTG | 0.08 | 0.71 | 0.17 | 0.23 | 0.24 | 0.15 |
| | ATTT | 0.36 | 10.26 | 1.72 | 1.28 | 1.95 | 1.56 |
| | CAAA | 0.66 | 9.81 | 10.95 | 5.52 | 2.50 | 2.26 |
| | CAAC | 0.21 | 3.35 | 3.84 | 1.35 | 0.44 | 0.76 |
| | CAAG | 0.20 | 4.22 | 4.65 | 1.74 | 1.13 | 0.78 |
| | CAAT | 0.17 | 3.00 | 4.00 | 1.42 | 0.45 | 0.64 |
| | CACA | 0.40 | 11.03 | 10.86 | 4.61 | 2.47 | 2.26 |
| | CACC | 0.06 | 1.59 | 2.28 | 1.01 | 0.28 | 0.57 |
| | CACG | 0.17 | 3.82 | 4.43 | 1.60 | 0.90 | 1.01 |
| | CACT | 0.30 | 5.19 | 7.15 | 2.84 | 1.24 | 1.23 |
| | CAGA | 0.34 | 6.16 | 5.67 | 2.44 | 1.13 | 1.56 |
| | CAGC | 0.07 | 0.62 | 1.20 | 0.37 | 0.23 | 0.24 |
| | CAGG | 0.14 | 4.62 | 2.33 | 1.23 | 0.56 | 0.61 |
| | CAGT | 0.08 | 2.23 | 1.34 | 0.45 | 0.25 | 0.31 |
| | CATA | 0.16 | 3.81 | 2.10 | 0.99 | 0.38 | 0.60 |
| | CATC | 0.06 | 1.21 | 0.87 | 0.31 | 0.18 | 0.33 |
| | CATG | 0.04 | 0.85 | 0.32 | 0.21 | 0.14 | 0.09 |
| | CATT | 0.03 | 1.80 | 0.25 | 0.13 | 0.08 | 0.07 |
| | CCAA | 0.66 | 10.16 | 7.47 | 4.54 | 1.51 | 1.80 |
| | CCAC | 0.38 | 6.09 | 6.42 | 3.54 | 1.12 | 1.71 |
| | CCAG | 0.49 | 15.75 | 4.57 | 2.71 | 1.07 | 1.17 |
| | CCAT | 0.24 | 4.01 | 2.72 | 1.71 | 0.55 | 0.71 |
| | CCCA | 0.72 | 14.19 | 9.83 | 6.35 | 2.42 | 2.77 |
| | CCCC | 0.44 | 7.69 | 6.61 | 4.00 | 1.13 | 2.26 |

**[Table 19]**

| Number | Base sequence (5'→3') | 5' adapter (SEQ ID NO: 1) | 5' adapter (SEQ ID NO: 2) | 5' adapter (SEQ ID NO: 3) | 5' adapter (SEQ ID NO: 4) | 5' adapter (SEQ ID NO: 5) | 5' adapter (SEQ ID NO: 6) |
|---|---|---|---|---|---|---|---|
| | CCCG | 0.48 | 6.92 | 4.60 | 3.04 | 0.99 | 1.75 |
| | CCCT | 0.36 | 6.21 | 3.90 | 2.37 | 0.92 | 1.26 |
| | CCGA | 0.26 | 3.79 | 2.54 | 1.56 | 0.45 | 0.72 |
| | CCGC | 0.15 | 1.57 | 1.51 | 0.81 | 0.28 | 0.57 |
| | CCGG | 0.26 | 3.45 | 1.51 | 0.85 | 0.42 | 0.76 |
| | CCGT | 0.10 | 1.35 | 0.80 | 0.48 | 0.18 | 0.40 |
| | CCTA | 0.17 | 2.34 | 1.31 | 1.07 | 0.31 | 0.38 |
| | CCTC | 0.12 | 1.81 | 1.14 | 0.78 | 0.32 | 0.42 |
| | CCTG | 0.26 | 3.04 | 2.15 | 1.26 | 0.64 | 0.59 |
| | CCTT | 0.09 | 1.46 | 0.96 | 0.70 | 0.24 | 0.26 |
| | CGAA | 0.84 | 12.11 | 11.38 | 3.56 | 2.00 | 2.63 |
| | CGAC | 0.15 | 2.98 | 2.83 | 0.98 | 0.48 | 0.60 |
| | CGAG | 0.31 | 4.73 | 5.41 | 1.66 | 1.22 | 0.98 |
| | CGAT | 0.10 | 1.24 | 1.45 | 0.54 | 0.24 | 0.18 |
| | CGCA | 0.31 | 4.21 | 7.70 | 2.00 | 1.03 | 1.22 |
| | CGCC | 0.09 | 1.35 | 2.39 | 0.57 | 0.35 | 0.41 |
| | CGCG | 0.23 | 3.08 | 5.05 | 1.20 | 0.70 | 1.03 |
| | CGCT | 0.23 | 3.39 | 5.72 | 1.72 | 0.62 | 0.93 |
| | CGGA | 0.66 | 10.64 | 8.41 | 2.84 | 1.76 | 1.93 |
| | CGGC | 0.16 | 1.94 | 3.15 | 0.84 | 0.43 | 0.57 |
| | CGGG | 0.41 | 6.13 | 4.04 | 1.60 | 0.98 | 1.72 |
| | CGGT | 0.08 | 1.00 | 0.79 | 0.38 | 0.17 | 0.21 |
| | CGTA | 0.23 | 3.85 | 3.37 | 1.42 | 0.82 | 0.81 |
| | CGTC | 0.04 | 0.51 | 0.33 | 0.16 | 0.24 | 0.15 |
| | CGTG | 0.12 | 1.07 | 0.85 | 0.40 | 0.37 | 0.18 |
| | CGTT | 0.15 | 2.45 | 2.08 | 0.73 | 0.80 | 0.43 |
| | CTAA | 0.30 | 4.80 | 2.43 | 1.64 | 0.82 | 0.88 |
| | CTAC | 0.09 | 1.93 | 1.04 | 0.70 | 0.30 | 0.40 |
| | CTAG | 0.14 | 2.56 | 1.21 | 0.88 | 0.58 | 0.51 |
| | CTAT | 0.04 | 1.07 | 0.52 | 0.38 | 0.10 | 0.21 |
| | CTCA | 0.30 | 6.18 | 2.95 | 1.74 | 0.89 | 1.09 |
| | CTCC | 0.15 | 2.18 | 1.32 | 0.90 | 0.36 | 0.66 |
| | CTCG | 0.35 | 4.30 | 2.40 | 1.68 | 0.90 | 1.28 |
| | CTCT | 0.34 | 4.36 | 2.15 | 1.76 | 0.84 | 1.18 |
| | CTGA | 0.27 | 3.82 | 2.14 | 1.93 | 0.81 | 1.09 |
| | CTGC | 0.07 | 0.73 | 0.46 | 0.38 | 0.16 | 0.28 |
| | CTGG | 0.17 | 1.97 | 0.80 | 0.68 | 0.40 | 0.51 |
| | CTGT | 0.12 | 1.05 | 0.60 | 0.48 | 0.27 | 0.38 |
| | CTTA | 0.10 | 1.87 | 0.65 | 0.45 | 0.28 | 0.37 |
| | CTTC | 0.08 | 1.22 | 0.59 | 0.41 | 0.22 | 0.34 |
| | CTTG | 0.07 | 1.32 | 0.43 | 0.41 | 0.31 | 0.27 |
| | CTTT | 0.04 | 1.37 | 0.30 | 0.22 | 0.13 | 0.17 |
| 20 | GAAA | 7.06 | 9.99 | 23.23 | 8.00 | 1.50 | 6.93 |

**[Table 20]**

| Number | Base sequence (5'→3') | 5' adapter (SEQ ID NO: 1) | 5' adapter (SEQ ID NO: 2) | 5' adapter (SEQ ID NO: 3) | 5' adapter (SEQ ID NO: 4) | 5' adapter (SEQ ID NO: 5) | 5' adapter (SEQ ID NO: 6) |
|---|---|---|---|---|---|---|---|
| | GAAC | 4.84 | 5.53 | 13.87 | 1.49 | 0.23 | 6.60 |
| 58 | GAAG | 1.59 | 2.15 | 7.92 | 7.86 | 1.14 | 2.00 |
| | GAAT | 2.34 | 4.43 | 8.30 | 3.43 | 0.75 | 2.32 |
| 59 | GACA | 4.44 | 4.68 | 14.10 | 7.31 | 1.41 | 4.73 |
| 60 | GACC | 1.87 | 2.47 | 6.71 | 3.68 | 1.36 | 4.10 |
| | GACG | 2.13 | 1.78 | 5.71 | 4.27 | 0.63 | 2.66 |
| | GACT | 2.39 | 3.26 | 5.38 | 4.13 | 0.94 | 2.11 |
| 21 | GAGA | 3.66 | 3.32 | 11.91 | 5.88 | 1.79 | 3.49 |
| | GAGC | 0.51 | 0.07 | 2.56 | 1.19 | 0.50 | 0.62 |
| | GAGG | 2.14 | 1.23 | 5.20 | 6.72 | 0.69 | 0.96 |
| | GAGT | 0.67 | 0.75 | 1.80 | 1.06 | 0.23 | 0.50 |
| | GATA | 0.54 | 2.63 | 2.62 | 2.09 | 0.44 | 0.70 |
| | GATC | 0.47 | 1.52 | 1.65 | 0.76 | 0.23 | 0.62 |
| | GATG | 0.51 | 1.69 | 1.80 | 1.67 | 0.54 | 0.49 |
| | GATT | 0.43 | 3.94 | 1.04 | 1.14 | 0.32 | 0.53 |
| 72 | GCAA | 1.31 | 5.04 | 8.65 | 3.53 | 1.05 | 2.34 |
| | GCAC | 0.36 | 1.07 | 3.47 | 1.43 | 0.25 | 1.05 |
| 73 | GCAG | 1.29 | 4.61 | 9.42 | 3.81 | 1.18 | 2.73 |
| | GCAT | 0.46 | 2.60 | 3.01 | 1.26 | 0.33 | 0.99 |
| | GCCA | 0.67 | 2.62 | 2.91 | 1.90 | 0.56 | 1.28 |
| | GCCC | 0.43 | 2.61 | 3.53 | 1.56 | 0.66 | 1.74 |
| | GCCG | 0.32 | 0.88 | 1.15 | 1.01 | 0.19 | 0.54 |
| | GCCT | 0.19 | 1.00 | 1.31 | 0.57 | 0.36 | 0.48 |
| | GCGA | 0.88 | 5.96 | 7.58 | 2.05 | 0.71 | 2.04 |
| | GCGC | 0.41 | 0.57 | 1.69 | 0.59 | 0.19 | 0.73 |
| | GCGG | 0.74 | 2.51 | 2.33 | 1.05 | 0.29 | 1.19 |
| | GCGT | 0.34 | 1.42 | 1.23 | 0.47 | 0.16 | 0.46 |
| | GCTA | 0.53 | 2.54 | 2.70 | 1.43 | 0.42 | 0.93 |
| | GCTC | 0.66 | 2.26 | 3.11 | 1.99 | 0.55 | 1.54 |
| | GCTG | 0.59 | 1.26 | 2.33 | 1.02 | 0.38 | 0.46 |
| | GCTT | 0.33 | 1.65 | 1.65 | 0.86 | 0.25 | 0.53 |
| 22 | GGAA | 8.64 | 10.55 | 21.15 | 6.16 | 3.08 | 7.01 |
| 61 | GGAC | 3.34 | 3.45 | 9.78 | 5.18 | 1.25 | 3.85 |
| 23 | GGAG | 3.11 | 2.48 | 8.97 | 5.94 | 1.91 | 2.74 |
| | GGAT | 1.29 | 2.68 | 3.27 | 1.52 | 0.49 | 0.93 |
| 62 | GGCA | 2.95 | 1.08 | 12.09 | 4.81 | 1.60 | 3.72 |
| | GGCC | 2.06 | 0.66 | 5.76 | 2.59 | 1.62 | 1.97 |
| | GGCG | 2.45 | 0.84 | 6.44 | 3.21 | 1.31 | 3.33 |
| 63 | GGCT | 3.12 | 1.66 | 5.31 | 3.27 | 1.47 | 2.27 |
| 24 | GGGA | 4.50 | 5.88 | 11.60 | 6.02 | 2.34 | 3.79 |
| | GGGC | 0.99 | 0.34 | 3.25 | 1.82 | 0.80 | 1.57 |
| 64 | GGGG | 3.16 | 3.58 | 6.33 | 3.98 | 1.22 | 56.99 |
| | GGGT | 0.94 | 1.33 | 1.53 | 0.90 | 0.28 | 0.53 |

**[Table 21]**

| Number | Base sequence (5'→3') | 5' adapter (SEQ ID NO: 1) | 5' adapter (SEQ ID NO: 2) | 5' adapter (SEQ ID NO: 3) | 5' adapter (SEQ ID NO: 4) | 5' adapter (SEQ ID NO: 5) | 5' adapter (SEQ ID NO: 6) |
|---|---|---|---|---|---|---|---|
| | GGTA | 1.90 | 3.36 | 3.49 | 2.27 | 0.74 | 1.15 |
| | GGTC | 0.29 | 0.74 | 0.49 | 0.51 | 0.17 | 0.35 |
| | GGTG | 0.89 | 1.93 | 2.56 | 2.50 | 0.54 | 0.93 |
| | GGTT | 1.10 | 2.03 | 2.91 | 1.63 | 0.94 | 0.92 |
| 74 | GTAA | 1.01 | 4.86 | 5.61 | 3.98 | 1.14 | 2.27 |
| | GTAC | 0.77 | 2.52 | 3.04 | 2.77 | 0.65 | 1.66 |
| 65 | GTAG | 1.43 | 2.21 | 5.28 | 4.91 | 2.10 | 2.40 |
| | GTAT | 0.22 | 1.25 | 1.03 | 0.50 | 0.21 | 0.30 |
| 75 | GTCA | 1.41 | 6.45 | 5.38 | 4.81 | 1.34 | 2.74 |
| | GTCC | 0.87 | 2.92 | 3.19 | 2.95 | 1.33 | 2.10 |
| 25 | GTCG | 1.56 | 1.73 | 2.69 | 2.81 | 1.75 | 1.51 |
| | GTCT | 0.84 | 2.57 | 2.68 | 1.99 | 0.65 | 1.42 |
| | GTGA | 0.50 | 2.32 | 2.35 | 2.10 | 0.54 | 1.24 |
| | GTGC | 0.22 | 0.50 | 0.79 | 0.70 | 0.26 | 0.62 |
| | GTGG | 0.47 | 1.53 | 1.90 | 2.29 | 0.54 | 1.12 |
| | GTGT | 0.23 | 0.81 | 0.77 | 0.74 | 0.22 | 0.49 |
| | GTTA | 0.24 | 1.17 | 0.50 | 0.56 | 0.26 | 0.30 |
| | GTTC | 0.28 | 1.33 | 0.88 | 0.79 | 0.11 | 0.59 |
| | GTTG | 0.71 | 0.89 | 1.08 | 1.98 | 0.48 | 0.40 |
| | GTTT | 0.52 | 3.68 | 2.59 | 2.14 | 0.96 | 2.57 |
| 26 | TAAA | 6.70 | 36.52 | 26.40 | 17.75 | 8.95 | 8.28 |
| 27 | TAAC | 2.39 | 18.62 | 14.79 | 5.13 | 3.06 | 4.19 |
| 28 | TAAG | 2.87 | 14.40 | 12.06 | 9.65 | 6.47 | 3.64 |
| | TAAT | 0.64 | 15.29 | 4.93 | 2.69 | 0.83 | 1.37 |
| 29 | TACA | 6.02 | 33.92 | 21.59 | 15.89 | 7.61 | 6.43 |
| 30 | TACC | 1.50 | 12.65 | 8.46 | 5.36 | 3.06 | 3.31 |
| 31 | TACG | 3.18 | 16.28 | 7.91 | 5.21 | 2.38 | 2.74 |
| | TACT | 1.46 | 13.51 | 4.15 | 4.72 | 0.85 | 1.45 |
| 32 | TAGA | 4.89 | 14.77 | 19.09 | 9.27 | 6.36 | 5.76 |
| | TAGC | 0.61 | 0.67 | 1.98 | 1.21 | 0.48 | 0.64 |
| 33 | TAGG | 1.76 | 10.10 | 5.45 | 3.72 | 2.49 | 2.57 |
| | TAGT | 0.48 | 6.09 | 2.98 | 1.73 | 1.13 | 0.95 |
| | TATA | 0.77 | 11.41 | 1.52 | 1.34 | 0.64 | 0.39 |
| | TATC | 0.47 | 2.64 | 0.82 | 0.73 | 0.12 | 0.44 |
| | TATG | 0.23 | 1.82 | 0.28 | 0.49 | 0.16 | 0.11 |
| 34 | TATT | 2.31 | 45.91 | 9.75 | 4.19 | 2.83 | 3.23 |
| 35 | TCAA | 7.44 | 33.38 | 16.85 | 14.69 | 6.73 | 6.52 |
| 66 | TCAC | 1.34 | 13.87 | 7.51 | 6.42 | 2.59 | 2.44 |
| 36 | TCAG | 2.44 | 25.78 | 10.73 | 10.72 | 4.12 | 4.73 |
| | TCAT | 0.50 | 12.53 | 2.39 | 1.35 | 0.36 | 0.66 |
| 37 | TCCA | 6.27 | 32.42 | 15.60 | 13.44 | 5.25 | 5.34 |
| 38 | TCCC | 2.68 | 21.23 | 7.84 | 6.88 | 2.98 | 4.10 |
| 39 | TCCG | 2.29 | 17.08 | 5.02 | 3.55 | 1.90 | 2.54 |

**[Table 22]**

| Number | Base sequence (5'→3') | 5' adapter (SEQ ID NO: 1) | 5' adapter (SEQ ID NO: 2) | 5' adapter (SEQ ID NO: 3) | 5' adapter (SEQ ID NO: 4) | 5' adapter (SEQ ID NO: 5) | 5' adapter (SEQ ID NO: 6) |
|---|---|---|---|---|---|---|---|
| 40 | TCCT | 2.72 | 17.27 | 5.59 | 5.94 | 1.59 | 2.18 |
| 41 | TCGA | 2.60 | 21.81 | 10.22 | 6.91 | 3.96 | 3.55 |
| | TCGC | 0.77 | 7.32 | 3.25 | 1.39 | 0.62 | 1.05 |
| 76 | TCGG | 2.23 | 7.29 | 2.32 | 1.48 | 1.03 | 2.54 |
| | TCGT | 0.78 | 12.59 | 1.80 | 0.99 | 0.77 | 0.95 |
| 77 | TCTA | 1.33 | 11.86 | 3.13 | 3.27 | 1.02 | 1.55 |
| 42 | TCTC | 7.45 | 20.75 | 20.14 | 18.43 | 5.48 | 9.34 |
| | TCTG | 1.11 | 7.18 | 1.99 | 2.65 | 0.69 | 0.61 |
| 43 | TCTT | 6.58 | 21.05 | 13.41 | 14.63 | 2.99 | 5.02 |
| 44 | TGAA | 10.04 | 25.29 | 32.08 | 14.95 | 4.17 | 7.34 |
| 67 | TGAC | 1.50 | 9.41 | 8.63 | 5.23 | 1.72 | 2.40 |
| 45 | TGAG | 3.47 | 7.54 | 14.09 | 6.94 | 3.02 | 2.72 |
| | TGAT | 0.94 | 4.92 | 3.35 | 2.99 | 1.74 | 0.65 |
| 46 | TGCA | 2.77 | 5.68 | 10.20 | 5.71 | 2.13 | 2.46 |
| | TGCC | 0.66 | 2.29 | 3.25 | 2.51 | 1.26 | 0.68 |
| 68 | TGCG | 1.50 | 3.29 | 6.94 | 3.53 | 1.18 | 2.10 |
| 69 | TGCT | 2.68 | 4.64 | 4.81 | 4.01 | 1.50 | 1.25 |
| 47 | TGGA | 5.74 | 12.23 | 16.74 | 9.46 | 3.68 | 5.25 |
| 70 | TGGC | 1.83 | 3.72 | 7.12 | 3.22 | 1.44 | 2.03 |
| 48 | TGGG | 4.02 | 8.08 | 7.91 | 4.70 | 2.86 | 3.55 |
| | TGGT | 0.99 | 3.01 | 1.86 | 1.42 | 0.67 | 0.73 |
| 49 | TGTA | 3.18 | 8.00 | 4.71 | 3.10 | 2.08 | 1.55 |
| | TGTC | 0.41 | 1.10 | 0.59 | 0.42 | 0.20 | 0.19 |
| | TGTG | 0.93 | 2.80 | 1.26 | 1.48 | 0.54 | 0.32 |
| | TGTT | 2.03 | 7.46 | 2.25 | 1.95 | 1.06 | 0.80 |
| | TTAA | 0.92 | 9.05 | 2.06 | 2.28 | 1.30 | 0.83 |
| | TTAC | 0.25 | 3.35 | 1.48 | 0.95 | 0.18 | 0.55 |
| | TTAG | 0.45 | 4.21 | 1.78 | 1.85 | 1.27 | 0.84 |
| 71 | TTAT | 1.71 | 21.39 | 6.59 | 5.20 | 4.11 | 1.49 |
| | TTCA | 1.10 | 11.68 | 2.39 | 2.79 | 0.85 | 1.34 |
| | TTCC | 0.27 | 5.89 | 1.86 | 2.37 | 1.29 | 0.81 |
| | TTCG | 0.57 | 3.49 | 1.62 | 1.26 | 0.76 | 0.93 |
| 50 | TTCT | 1.97 | 16.78 | 4.46 | 3.85 | 2.81 | 2.79 |
| | TTGA | 0.57 | 3.41 | 1.59 | 1.43 | 0.85 | 0.66 |
| | TTGC | 0.16 | 0.90 | 0.54 | 0.32 | 0.25 | 0.23 |
| | TTGG | 0.37 | 2.27 | 0.93 | 0.99 | 0.62 | 0.53 |
| | TTGT | 0.15 | 1.49 | 0.32 | 0.43 | 0.13 | 0.23 |
| 51 | TTTA | 4.44 | 26.06 | 7.29 | 7.81 | 9.09 | 4.76 |
| 52 | TTTC | 4.20 | 19.53 | 5.56 | 5.56 | 2.51 | 2.83 |
| | TTTG | 0.33 | 4.83 | 0.39 | 0.61 | 0.20 | 0.25 |
| 53 | TTTT | 1.69 | 23.46 | 5.02 | 4.63 | 5.51 | 3.23 |

Table 15 summarizes the 3' adapters (78 kinds) that had an effective read rate of 1.0% or more in common for all 6 kinds of 5' adapters. When an effective read rate of 1.0% or more is obtained, for example, even when a small sequencer Miniseq manufactured by Illumina, Inc. is used, about 250,000 effective reads can be obtained, that is, it can be said that this is a numerical value of effective reads that enables a certain level of analysis. Therefore, it can be said that the 3' adapters having an effective read rate of 1.0% or more are practically excellent in sequencing efficiency.

**[Table 23]**

| Number | Base sequence (5'→3') | Number | Base sequence (5'→3') | Number | Base sequence (5'→3') | Number | Base sequence (5'→3') |
|---|---|---|---|---|---|---|---|
| 1 | AAAA | 21 | GAGA | 41 | TCGA | 61 | GGAC |
| 2 | AAAC | 22 | GGAA | 42 | TCTC | 62 | GGCA |
| 3 | AAAG | 23 | GGAG | 43 | TCTT | 63 | GGCT |
| 4 | AAAT | 24 | GGGA | 44 | TGAA | 64 | GGGG |
| 5 | AACA | 25 | GTCG | 45 | TGAG | 65 | GTAG |
| 6 | AACG | 26 | TAAA | 46 | TGCA | 66 | TCAC |
| 7 | AACT | 27 | TAAC | 47 | TGGA | 67 | TGAC |
| 8 | AAGA | 28 | TAAG | 48 | TGGG | 68 | TGCG |
| 9 | AATT | 29 | TACA | 49 | TGTA | 69 | TGCT |
| 10 | ACAA | 30 | TACC | 50 | TTCT | 70 | TGGC |
| 11 | AGAA | 31 | TACG | 51 | TTTA | 71 | TTAT |
| 12 | AGAG | 32 | TAGA | 52 | TTTC | 72 | GCAA |
| 13 | AGCA | 33 | TAGG | 53 | TTTT | 73 | GCAG |
| 14 | AGCG | 34 | TATT | 54 | AACC | 74 | GTAA |
| 15 | AGCT | 35 | TCAA | 55 | AAGG | 75 | GTCA |
| 16 | AGGA | 36 | TCAG | 56 | ACTT | 76 | TCGG |
| 17 | AGGC | 37 | TCCA | 57 | AGTT | 77 | TCTA |
| 18 | AGGG | 38 | TCCC | 58 | GAAG | 78 | AGAT |
| 19 | AGTA | 39 | TCCG | 59 | GACA | | |
| 20 | GAAA | 40 | TCCT | 60 | GACC | | |

Table 16 summarizes the 3' adapters (53 kinds) that had an effective read rate of 1.5% or more in common for all 6 kinds of 5' adapters.

**[Table 24]**

| Number | Base sequence (5'→3') | Number | Base sequence (5'→3') | Number | Base sequence (5'→3') |
|---|---|---|---|---|---|
| 1 | AAAA | 19 | AGTA | 37 | TCCA |
| 2 | AAAC | 20 | GAAA | 38 | TCCC |
| 3 | AAAG | 21 | GAGA | 39 | TCCG |
| 4 | AAAT | 22 | GGAA | 40 | TCCT |
| 5 | AACA | 23 | GGAG | 41 | TCGA |
| 6 | AACG | 24 | GGGA | 42 | TCTC |
| 7 | AACT | 25 | GTCG | 43 | TCTT |
| 8 | AAGA | 26 | TAAA | 44 | TGAA |
| 9 | AATT | 27 | TAAC | 45 | TGAG |
| 10 | ACAA | 28 | TAAG | 46 | TGCA |
| 11 | AGAA | 29 | TACA | 47 | TGGA |
| 12 | AGAG | 30 | TACC | 48 | TGGG |
| 13 | AGCA | 31 | TACG | 49 | TGTA |
| 14 | AGCG | 32 | TAGA | 50 | TTCT |
| 15 | AGCT | 33 | TAGG | 51 | TTTA |
| 16 | AGGA | 34 | TATT | 52 | TTTC |
| 17 | AGGC | 35 | TCAA | 53 | TTTT |
| 18 | AGGG | 36 | TCAG | | |

Table 17 summarizes the 3' adapters (37 kinds) that had an effective read rate of 2.0% or more in common for all 6 kinds of 5' adapters. When an effective read rate of 2.0% or more is obtained, for example, even when a small sequencer Miniseq manufactured by Illumina, Inc. is used, about 500,000 effective reads can be obtained, that is, it can be said that this is a numerical value of effective reads that enables highly accurate analysis at a sufficient level. Therefore, it can be said that the 3' adapters having an effective read rate of 2.0% or more are practically particularly excellent in sequencing efficiency.

**[Table 25]**

| Number | Base sequence (5'→3') | Number | Base sequence (5'→3') | Number | Base sequence (5'→3') |
|---|---|---|---|---|---|
| 1 | AAAA | 18 | AGGG | 38 | TCCC |
| 2 | AAAC | 22 | GGAA | 41 | TCGA |
| 3 | AAAG | 24 | GGGA | 42 | TCTC |
| 5 | AACA | 26 | TAAA | 43 | TCTT |
| 6 | AACG | 27 | TAAC | 44 | TGAA |
| 7 | AACT | 28 | TAAG | 45 | TGAG |
| 10 | ACAA | 29 | TACA | 46 | TGCA |
| 11 | AGAA | 31 | TACG | 47 | TGGA |
| 12 | AGAG | 32 | TAGA | 48 | TGGG |
| 13 | AGCA | 34 | TATT | 51 | TTTA |
| 14 | AGCG | 35 | TCAA | 52 | TTTC |
| 15 | AGCT | 36 | TCAG | | |
| 16 | AGGA | 37 | TCCA | | |

Table 18 summarizes the 3' adapters (17 kinds) that had an effective read rate of 3.0% or more in common for all 6 kinds of 5' adapters.

**[Table 26]**

| Number | Base sequence (5'→3') | Number | Base sequence (5'→3') |
|---|---|---|---|
| 1 | AAAA | 29 | TACA |
| 3 | AAAG | 32 | TAGA |
| 5 | AACA | 35 | TCAA |
| 11 | AGAA | 37 | TCCA |
| 12 | AGAG | 42 | TCTC |
| 16 | AGGA | 44 | TGAA |
| 18 | AGGG | 47 | TGGA |
| 22 | GGAA | 51 | TTTA |
| 26 | TAAA | | |

Table 19 summarizes the 3' adapters (8 kinds) that had an effective read rate of 5.0% or more in common for all 6 kinds of 5' adapters.

**[Table 27]**

| Number | Base sequence (5'→3') |
|---|---|
| 5 | AACA |
| 11 | AGAA |
| 16 | AGGA |
| 26 | TAAA |
| 29 | TACA |
| 35 | TCAA |
| 37 | TCCA |
| 42 | TCTC |

### (Correlation of Effective Read Rate of Each 3' Adapter between Different Kinds of 5' Adapters)

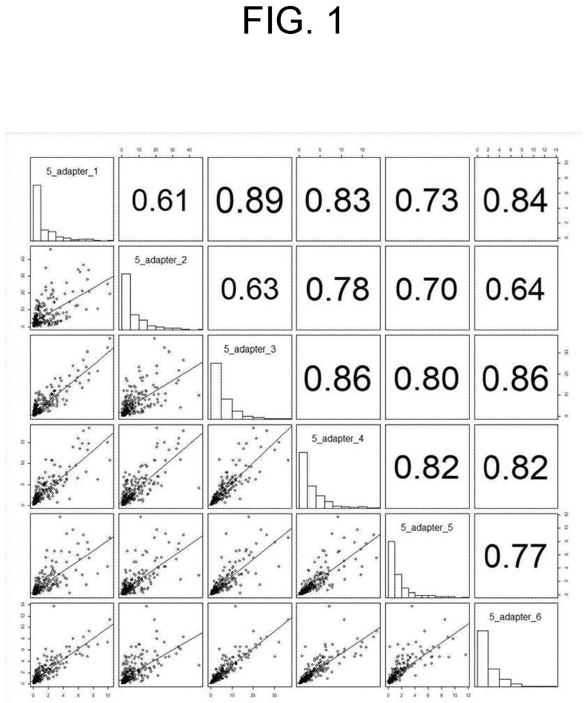

Plotting was performed with the X-axis indicating the effective read rate of each 3' adapter when a certain 5' adapter was used and the Y-axis indicating the effective read rate of each 3' adapter when another 5' adapter was used, and the correlation coefficient was calculated. The results are shown in Fig. 1.

Among the 256 kinds of 3' adapters, only the effective read rate value of the 3' adapter in which the base sequence of the 5' end excluding the adenylic acid at the 5' end is "GGGG" was an extreme outlier. Therefore, it was excluded from the calculation of the correlation coefficient and from Fig. 1.

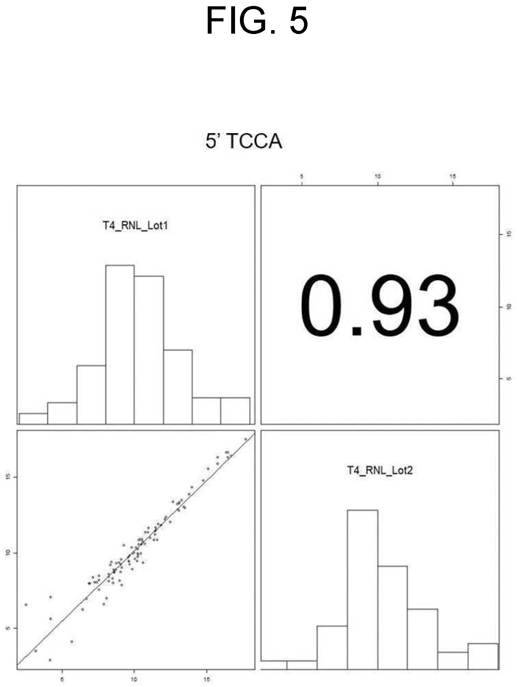

In Fig. 1, 5_adapter_1, 5_adapter_2, 5_adapter_3, 5_adapter_4, 5_adapter_5, and 5_adapter_6 mean a 5' adapter (SEQ ID NO: 1), a 5' adapter (SEQ ID NO: 2), a 5' adapter (SEQ ID NO: 3), a 5' adapter (SEQ ID NO: 4), a 5' adapter (SEQ ID NO: 5), and a 5' adapter (SEQ ID NO: 6), respectively.

When the calculation of the correlation coefficient was performed for all combinations (selecting 2 kinds of 5' adapters from 6 kinds of 5' adapters; ₆C₂ = 15 combinations), all the correlation coefficients were 0.6 or more, and the effective read rate of each 3' adapter between different kinds of 5' adapters showed a positive correlation. That is, it was found that, regardless of the kind of 5' adapter (i.e., the base sequence of the 5' adapter), superior 3' adapters are commonly superior among the 3' adapters according to the present disclosure.

Example 2 (Embodiment B): Relationship between Base Sequences of 3' Adapter and 5' Adapter and Lot-to-lot Variation of T4 RNA Ligase 1

In preparing an NGS library, when the ligation of a 3' adapter and a 5' adapter to the target nucleic acids is performed using T4 RNA Ligase 1, lot-to-lot variation occurs. First, it was investigated which of the ligation of the 3' adapter and the ligation of the 5' adapter is more strongly influenced by the lot-to-lot variation of T4 RNA Ligase 1.

As a 3' adapter, a 5'-adenylated single-stranded DNA of 5'App/AACTGTAGGCACCATCAAT/3'ddC (SEQ ID NO: 11) was purchased. The 3' end of the 3' adapter is blocked by dideoxycytidine modification.

As a 5' adapter, a single-stranded RNA of 5'-GUUCAGAGUUCUACAGUCCGACGAUC-3' (SEQ ID NO: 4) was purchased.

As T4 RNA Ligase 1, two kinds of T4 RNA Ligase 1 (New England Biolabs Inc.) of different lots were prepared. Hereinafter, the two kinds of T4 RNA Ligase 1 are referred to as T4 RNA Ligase 1 (1) and T4 RNA Ligase 1 (2), respectively.

### <Preparation of NGS Library>

Using the two kinds of T4 RNA Ligase 1, an NGS library was prepared by the following procedure under each of the following conditions (A) to (C).
(A) The two kinds of T4 RNA Ligase 1 are used in both the ligation of the 3' adapter and the ligation of the 5' adapter
(B) The two kinds of T4 RNA Ligase 1 are used only in the ligation of the 3' adapter, and T4 RNA Ligase 1 (1) is used in the ligation of the 5' adapter
(C) The two kinds of T4 RNA Ligase 1 are used only in the ligation of the 5' adapter, and T4 RNA Ligase 1(1) is used in the ligation of the 3' adapter

Using the 3' adapter and the 5' adapter respectively, an NGS library of small RNAs (in this Example, human-derived serum RNA was used) was prepared. The NGS library was prepared using a method with reference to Seda Eminaga et al., Quantification of microRNA Expression with Next-Generation Sequencing, Curr Protoc Mol Biol. 2013 July; 04:Unit 4.17. That is, operations of [1] ligation of the 3' adapter, [2] annealing of a reverse transcription primer, [3] ligation of the 5' adapter, [4] reverse transcription, [5] PCR, and [6] purification of the PCR product were performed.
[1] Ligation of the 3' adapter
   Nuclease free water 1.0 µL
   T4 RNA Ligase Reaction Buffer (NEB/M0242) 1.0 µL
   100% DMSO (13445-74/Nacalai Tesque) 1.0 µL
   50% PEG (NEB/B1004) 1.0 µL
   Total RNA (human serum-derived RNA) 4.0 µL
   To a mix of the above, 10 µM 3' adapter was added, and the mixture was incubated at 90°C for 30 seconds.
   T4 RNA Ligase 1 (NEB/0204M) 1.5 µL
   SUPERase inhibitor (Thermo Fisher/AM2694) 0.5 µL

Further, the above-described mix was added, and the total RNA and the 3' adapter were ligated by incubation at 22°C for 60 minutes.

### [2] Annealing of an RT primer

To the solution obtained by the operation of [1], 1 µL of a 10 µM RT primer was added, and the mixture was incubated at 90°C for 30 seconds, and further incubated at 65°C for 5 minutes.

The base sequence of the RT primer is shown in SEQ ID NO: 8 below. The portion represented by N is a UMI sequence. In the following sequence, N each independently represents A, T, G, or C.
(SEQ ID NO: 8) 5'-GTGACTGGAGTTCAGACGTGTGCTCTTCCGATCTNNNNNNNNNNNNATTGATGGT GCCTAC-3'

### [3] Ligation of the 5' adapter

Nuclease free water 0.8 µL
ATP (NEB/0204M) 1.4 µL
T4 RNA Ligase 1 (NEB/0204M) 1.5 µL

To the solution obtained by the operation of [2], a 10 µM 5' adapter was respectively added to the above-described mix, and the mixture was incubated at 20°C for 60 minutes.

### [4] Reverse transcription

Nuclease free water 3.5 µL
25 mM dNTP mix (Nippon Gene/312-07271) 0.3 µL
5×FS Buffer (Thermo Fisher/18080093) 1.5 µL
0.1M DTT (Thermo Fisher/18080093) 1.5 µL
RNase OUT (Thermo Fisher/10777019) 0.375 µL
SuperScript III (Thermo Fisher/18080093) 1.5 µL

Further, to 7.5 µL of the solution obtained by the above-described incubation, the above-described mix was added, and the mixture was incubated at 48°C for 30 seconds and further incubated at 85°C for 5 minutes.

The base sequence of the RT primer is as shown in SEQ ID NO: 8 above.

### [5] PCR

Nuclease free water 24.1 µL
Phusion HF Buffer (NEB/M0530L) 10 µL
25 mM dNTP mix (Nippon Gene/312-07271) 0.4 µL
Phusion High-Fidelity DNA Polymerase (NEB/M0530L) 0.5 µL
10 µM PCR primer R 2.5 µL
10 µM PCR primer F 2.5 µL

To 10 µL of the solution obtained by the operation of [4], the above-described mix was added, and the mixture was incubated at 98°C for 30 seconds. Thereafter, PCR performing 18 cycles of a cycle of 98°C for 10 seconds, 60°C for 20 seconds, and 72°C for 20 seconds was performed.

The base sequence of the PCR primer R is shown in SEQ ID NO: 9 below.
(SEQ ID NO: 9) 5'-CAAGCAGAAGACGGCATACGAGATCGTGATGCTCCACCGATAAATATTAGCCCG T-3'

The base sequence of the PCR primer F is shown in SEQ ID NO: 10 below.
(SEQ ID NO: 10) 5'-AATGATACGGCGACCACCGAGATCTACACGTTCAGAGTTCTACAGTCCGA-3'

### <Sequencing>

The completed library was sequenced with Illumina Nextseq 550.

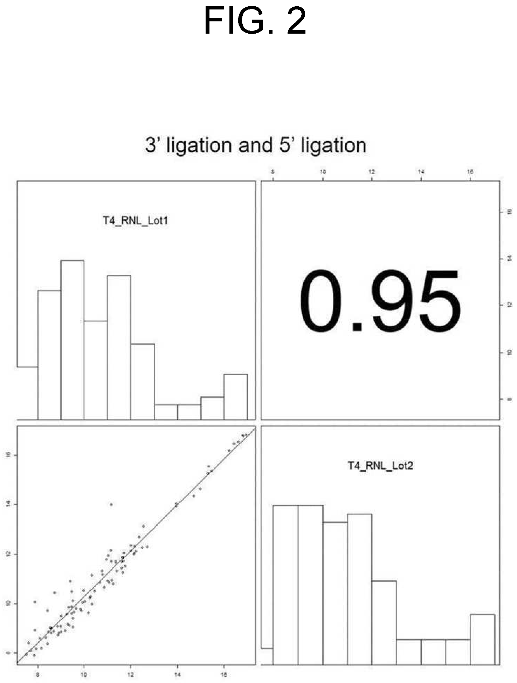

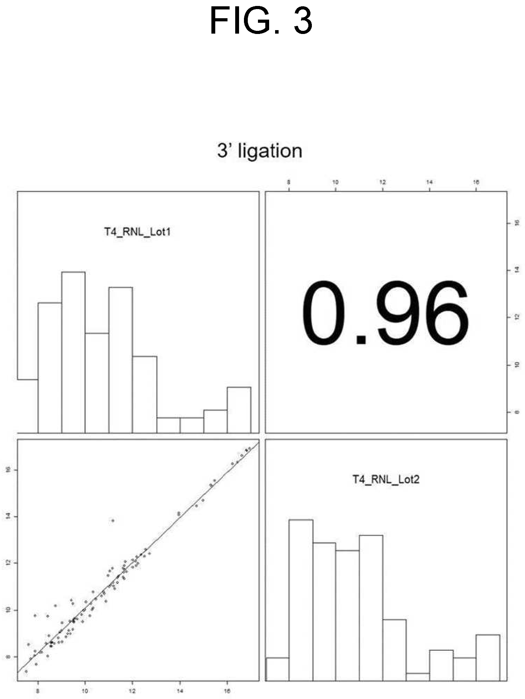

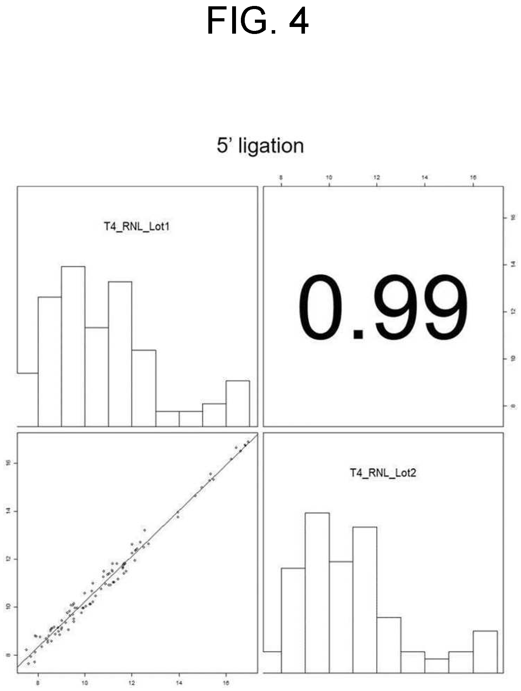

The coefficient of determination of the quantitative values of miRNAs having the top 100 expression levels when the two kinds of T4 RNA Ligase 1 were used was calculated to investigate the influence of the lot-to-lot variation. Plotting was performed with the X-axis indicating the quantitative value of miRNAs (average value of n = 4) when the NGS library was prepared using T4 RNA Ligase 1 (1) and the Y-axis indicating the quantitative value of the corresponding miRNAs (average value of n = 4) when the NGS library was prepared using T4 RNA Ligase 1 (2), and the coefficient of determination was calculated. As shown in Figs. 2 to 4, the coefficient of determination when (A) the two kinds of T4 RNA Ligase 1 were used in both the ligation of the 3' adapter and the ligation of the 5' adapter (Fig. 2) was 0.95, the coefficient of determination when (B) the two kinds of T4 RNA Ligase 1 were used only in the ligation of the 3' adapter (Fig. 3) was 0.96, and the coefficient of determination when (C) the two kinds of T4 RNA Ligase 1 were used only in the ligation of the 5' adapter (Fig. 4) was 0.99. Based on this, it was found that the influence of the lot difference was large in the ligation of the 3' adapter, and the influence of the lot difference was almost none in the ligation of the 5' adapter. This is believed to be because when T4 RNA Ligase 1 is used, the efficiency of the ligation of the 3' adapter is lower than that of the ligation of the 5' adapter, whereby the influence of the lot-to-lot variation was largely observed in the ligation of the 3' adapter. Therefore, in order to reduce the influence of the lot-to-lot difference, the base sequence of the 3' adapter was investigated.

### Example 3 (Embodiment B): Examination of Base Sequences of 3' Adapters Less Susceptible to Influence of Lot-to-lot Variation of T4 RNA Ligase 1

Next, 3' adapters less susceptible to the influence of the lot-to-lot variation of T4 RNA Ligase 1 were screened. From the sequences for which 1% or more of effective reads were obtained in Example 1, 15 kinds of sequences were used. Using the 15 kinds of 3' adapters and the 2 lots of T4 RNA Ligase 1, a total of 30 library preparations were performed, and the influence of the lot-to-lot variation of T4 RNA Ligase 1 when each 3' adapter was used was investigated. Since it is guaranteed from Example 2 that the lot-to-lot variation of T4 RNA Ligase 1 occurs in the ligation of 3' adapters, and from Example 1 that an effective read number of 1% or more is obtained in the 15 kinds of 3' adapters regardless of the sequence of the 5' adapter, the 5' adapter 4 (SEQ ID NO: 4) was used as the 5' adapter in this Example 3.

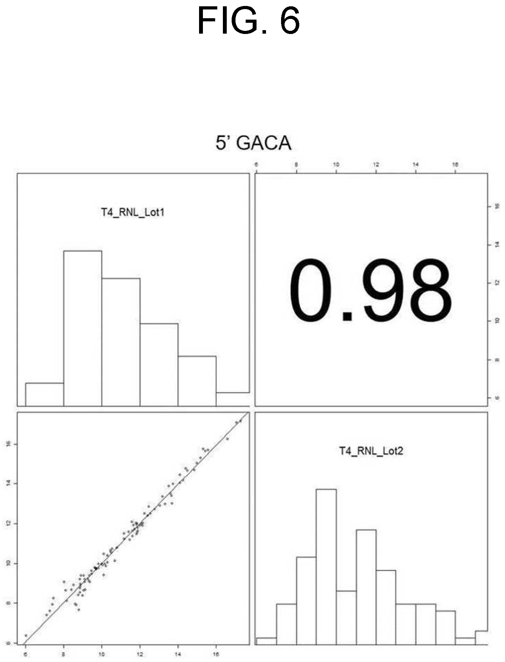

Similarly to Example 2, the coefficient of determination of the quantitative values of miRNAs having the top 100 expression levels when the two kinds of T4 RNA Ligase 1 were used was calculated to investigate the influence of the lot-to-lot variation. The following table shows the coefficient of determination when each 3' adapter is used. As shown in the following table and Fig. 5 and Fig. 6, it was found that, while there were 3' adapters strongly susceptible to the influence of the lot-to-lot variation of T4 RNA Ligase 1 (e.g., the 5' end is TCCA (Fig. 5); coefficient of determination is about 0.93), there were also adapter sequences less susceptible to the influence of the lot-to-lot variation of T4 RNA Ligase 1 (e.g., the 5' end is GACA (Fig. 6); coefficient of determination is about 0.98).

**[Table 28]**

| 5' terminal sequence of 3' adapter | Coefficient of determination |
|---|---|
| GACA | 0.980 |
| TCTT | 0.944 |
| ACAA | 0.969 |
| AACA | 0.977 |
| GTCA | 0.968 |
| TTAT | 0.961 |
| TGAA | 0.970 |
| TCTC | 0.953 |
| TACA | 0.942 |
| AACT | 0.963 |
| AGGA | 0.972 |
| TCCA | 0.926 |
| AACC | 0.953 |
| AACG | 0.970 |
| AGCT | 0.959 |

From the results of Examples 1 to 3, as 3' adapters (Embodiment B) capable of suppressing the formation of adapter dimers and the lot-to-lot variation of T4 RNA Ligase 1, 3' adapters having the following base sequences at the 5' end, in which the coefficient of determination between different lots is 0.960 or more, were determined to be suitable.

**[Table 29]**

| Table B | | |
|---|---|---|
| GACA | GTCA | AACT |
| ACAA | TTAT | AGGA |
| AACA | TGAA | AACG |

### (Appendix)

The means according to the present disclosure include the following aspects.
(1) A ligation method, including:
   ligating, to a 3' end of each of a plurality of kinds of nucleic acids having different sequences, a 5'-adenylated single-stranded DNA that is a 3' adapter in which a base sequence of a 5' end excluding an adenylic acid at the 5' end is any one of base sequences shown in the following Table A; and
   after the ligating of the 5'-adenylated single-stranded DNA, ligating, to a 5' end of each of the plurality of kinds of nucleic acids, a single-stranded nucleic acid that is a 5' adapter.

**[Table 30]**

| Table A | | | | | | | |
|---|---|---|---|---|---|---|---|
| Number | Base sequence (5'→3') | Number | Base sequence (5'→3') | Number | Base sequence (5'→3') | Number | Base sequence (5'→3') |
| 1 | AAAA | 21 | GAGA | 41 | TCGA | 61 | GGAC |
| 2 | AAAC | 22 | GGAA | 42 | TCTC | 62 | GGCA |
| 3 | AAAG | 23 | GGAG | 43 | TCTT | 63 | GGCT |
| 4 | AAAT | 24 | GGGA | 44 | TGAA | 64 | GGGG |
| 5 | AACA | 25 | GTCG | 45 | TGAG | 65 | GTAG |
| 6 | AACG | 26 | TAAA | 46 | TGCA | 66 | TCAC |
| 7 | AACT | 27 | TAAC | 47 | TGGA | 67 | TGAC |
| 8 | AAGA | 28 | TAAG | 48 | TGGG | 68 | TGCG |
| 9 | AATT | 29 | TACA | 49 | TGTA | 69 | TGCT |
| 10 | ACAA | 30 | TACC | 50 | TTCT | 70 | TGGC |
| 11 | AGAA | 31 | TACG | 51 | TTTA | 71 | TTAT |
| 12 | AGAG | 32 | TAGA | 52 | TTTC | 72 | GCAA |
| 13 | AGCA | 33 | TAGG | 53 | TTTT | 73 | GCAG |
| 14 | AGCG | 34 | TATT | 54 | AACC | 74 | GTAA |
| 15 | AGCT | 35 | TCAA | 55 | AAGG | 75 | GTCA |
| 16 | AGGA | 36 | TCAG | 56 | ACTT | 76 | TCGG |
| 17 | AGGC | 37 | TCCA | 57 | AGTT | 77 | TCTA |
| 18 | AGGG | 38 | TCCC | 58 | GAAG | 78 | AGAT |
| 19 | AGTA | 39 | TCCG | 59 | GACA | | |
| 20 | GAAA | 40 | TCCT | 60 | GACC | | |

(2) The ligation method according to (1), wherein the base sequence of the 5' end of the 3' adapter is any one selected from the group consisting of the base sequences of Numbers 1 to 53 shown in Table A above.

(3) The ligation method according to (1) or (2), wherein a ligase used for the ligation of the 3' adapter and a ligase used for the ligation of the 5' adapter are each independently at least one selected from the group consisting of T4 RNA Ligase 1, T4 RNA Ligase 2, T4 DNA ligase, and a variant thereof.

(4) A method of suppressing adapter dimer formation, the method including:
ligating, to a 3' end of each of a plurality of kinds of nucleic acids having different sequences, a 5'-adenylated single-stranded DNA that is a 3' adapter in which a base sequence of a 5' end, excluding an adenylic acid at the 5' end, is any one of base sequences shown in the following Table A; and
after the ligating of the 5'-adenylated single-stranded DNA, ligating, to a 5' end of each of the plurality of kinds of nucleic acids, a single-stranded nucleic acid that is a 5' adapter.

**[Table 31]**

| Table A | | | | | | | |
|---|---|---|---|---|---|---|---|
| Number | Base sequence (5'→3') | Number | Base sequence (5'→3') | Number | Base sequence (5'→3') | Number | Base sequence (5'→3') |
| 1 | AAAA | 21 | GAGA | 41 | TCGA | 61 | GGAC |
| 2 | AAAC | 22 | GGAA | 42 | TCTC | 62 | GGCA |
| 3 | AAAG | 23 | GGAG | 43 | TCTT | 63 | GGCT |
| 4 | AAAT | 24 | GGGA | 44 | TGAA | 64 | GGGG |
| 5 | AACA | 25 | GTCG | 45 | TGAG | 65 | GTAG |
| 6 | AACG | 26 | TAAA | 46 | TGCA | 66 | TCAC |
| 7 | AACT | 27 | TAAC | 47 | TGGA | 67 | TGAC |
| 8 | AAGA | 28 | TAAG | 48 | TGGG | 68 | TGCG |
| 9 | AATT | 29 | TACA | 49 | TGTA | 69 | TGCT |
| 10 | ACAA | 30 | TACC | 50 | TTCT | 70 | TGGC |
| 11 | AGAA | 31 | TACG | 51 | TTTA | 71 | TTAT |
| 12 | AGAG | 32 | TAGA | 52 | TTTC | 72 | GCAA |
| 13 | AGCA | 33 | TAGG | 53 | TTTT | 73 | GCAG |
| 14 | AGCG | 34 | TATT | 54 | AACC | 74 | GTAA |
| 15 | AGCT | 35 | TCAA | 55 | AAGG | 75 | GTCA |
| 16 | AGGA | 36 | TCAG | 56 | ACTT | 76 | TCGG |
| 17 | AGGC | 37 | TCCA | 57 | AGTT | 77 | TCTA |
| 18 | AGGG | 38 | TCCC | 58 | GAAG | 78 | AGAT |
| 19 | AGTA | 39 | TCCG | 59 | GACA | | |
| 20 | GAAA | 40 | TCCT | 60 | GACC | | |

(5) The method of suppressing adapter dimer formation according to (4), wherein the base sequence of the 5' end of the 3' adapter is any one selected from the group consisting of the base sequences of Numbers 1 to 53 shown in Table A above.

(6) The method of suppressing adapter dimer formation according to (4) or (5), wherein a ligase used for the ligation of the 3' adapter and a ligase used for the ligation of the 5' adapter are each independently at least one selected from the group consisting of T4 RNA Ligase 1, T4 RNA Ligase 2, T4 DNA ligase, and a variant thereof.

(7) A method of preparing an NGS library, the method including:
ligating, to a plurality of kinds of nucleic acids having different sequences, a 5'-adenylated single-stranded DNA that is a 3' adapter and a single-stranded nucleic acid that is a 5' adapter by the ligation method according to any one of (1) to (3);
synthesizing a cDNA by a reverse transcription reaction from the plurality of kinds of nucleic acids to which the 3' adapter and the 5' adapter have been ligated; and
amplifying the obtained cDNA by PCR.

(8) An NGS library preparation kit, including a 5'-adenylated single-stranded DNA contained in a first container, wherein a base sequence of a 5' end of the 5'-adenylated single-stranded DNA, excluding an adenylic acid at the 5' end, is any one of base sequences shown in the following Table A.

**[Table 32]**

| Table A | | | | | | | |
|---|---|---|---|---|---|---|---|
| Number | Base sequence (5'→3') | Number | Base sequence (5'→3') | Number | Base sequence (5'→3') | Number | Base sequence (5'→3') |
| 1 | AAAA | 21 | GAGA | 41 | TCGA | 61 | GGAC |
| 2 | AAAC | 22 | GGAA | 42 | TCTC | 62 | GGCA |
| 3 | AAAG | 23 | GGAG | 43 | TCTT | 63 | GGCT |
| 4 | AAAT | 24 | GGGA | 44 | TGAA | 64 | GGGG |
| 5 | AACA | 25 | GTCG | 45 | TGAG | 65 | GTAG |
| 6 | AACG | 26 | TAAA | 46 | TGCA | 66 | TCAC |
| 7 | AACT | 27 | TAAC | 47 | TGGA | 67 | TGAC |
| 8 | AAGA | 28 | TAAG | 48 | TGGG | 68 | TGCG |
| 9 | AATT | 29 | TACA | 49 | TGTA | 69 | TGCT |
| 10 | ACAA | 30 | TACC | 50 | TTCT | 70 | TGGC |
| 11 | AGAA | 31 | TACG | 51 | TTTA | 71 | TTAT |
| 12 | AGAG | 32 | TAGA | 52 | TTTC | 72 | GCAA |
| 13 | AGCA | 33 | TAGG | 53 | TTTT | 73 | GCAG |
| 14 | AGCG | 34 | TATT | 54 | AACC | 74 | GTAA |
| 15 | AGCT | 35 | TCAA | 55 | AAGG | 75 | GTCA |
| 16 | AGGA | 36 | TCAG | 56 | ACTT | 76 | TCGG |
| 17 | AGGC | 37 | TCCA | 57 | AGTT | 77 | TCTA |
| 18 | AGGG | 38 | TCCC | 58 | GAAG | 78 | AGAT |
| 19 | AGTA | 39 | TCCG | 59 | GACA | | |
| 20 | GAAA | 40 | TCCT | 60 | GACC | | |

(9) The NGS library preparation kit according to (8), wherein the base sequence of the 5' end of the 5'-adenylated single-stranded DNA is any one selected from the group consisting of the base sequences of Numbers 1 to 53 shown in Table A above.

(10) A 3' adapter for preparing a nucleic acid library, which is a single-stranded DNA in which a nucleotide at a 5' end is adenylated, wherein a base sequence of the 5' end of the single-stranded DNA, excluding an adenylic acid at the 5' end, is any one of base sequences shown in the following Table A.

**[Table 33]**

| Table A | | | | | | | |
|---|---|---|---|---|---|---|---|
| Number | Base sequence (5'→3') | Number | Base sequence (5'→3') | Number | Base sequence (5'→3') | Number | Base sequence (5'→3') |
| 1 | AAAA | 21 | GAGA | 41 | TCGA | 61 | GGAC |
| 2 | AAAC | 22 | GGAA | 42 | TCTC | 62 | GGCA |
| 3 | AAAG | 23 | GGAG | 43 | TCTT | 63 | GGCT |
| 4 | AAAT | 24 | GGGA | 44 | TGAA | 64 | GGGG |
| 5 | AACA | 25 | GTCG | 45 | TGAG | 65 | GTAG |
| 6 | AACG | 26 | TAAA | 46 | TGCA | 66 | TCAC |
| 7 | AACT | 27 | TAAC | 47 | TGGA | 67 | TGAC |
| 8 | AAGA | 28 | TAAG | 48 | TGGG | 68 | TGCG |
| 9 | AATT | 29 | TACA | 49 | TGTA | 69 | TGCT |
| 10 | ACAA | 30 | TACC | 50 | TTCT | 70 | TGGC |
| 11 | AGAA | 31 | TACG | 51 | TTTA | 71 | TTAT |
| 12 | AGAG | 32 | TAGA | 52 | TTTC | 72 | GCAA |
| 13 | AGCA | 33 | TAGG | 53 | TTTT | 73 | GCAG |
| 14 | AGCG | 34 | TATT | 54 | AACC | 74 | GTAA |
| 15 | AGCT | 35 | TCAA | 55 | AAGG | 75 | GTCA |
| 16 | AGGA | 36 | TCAG | 56 | ACTT | 76 | TCGG |
| 17 | AGGC | 37 | TCCA | 57 | AGTT | 77 | TCTA |
| 18 | AGGG | 38 | TCCC | 58 | GAAG | 78 | AGAT |
| 19 | AGTA | 39 | TCCG | 59 | GACA | | |
| 20 | GAAA | 40 | TCCT | 60 | GACC | | |

(11) The 3' adapter for preparing a nucleic acid library according to (10), wherein the base sequence of the 5' end is any one selected from the group consisting of the base sequences of Numbers 1 to 53 shown in Table A above.

The means according to Embodiment B of the present disclosure includes the following aspects.
(1) A ligation method, including:
   ligating, to a 3' end of each of a plurality of kinds of nucleic acids having different sequences, a 5'-adenylated single-stranded DNA that is a 3' adapter in which a base sequence of a 5' end, excluding an adenylic acid at the 5' end, is any one of base sequences shown in the following Table B; and
   after the ligating of the 5'-adenylated single-stranded DNA, ligating, to a 5' end of each of the plurality of kinds of nucleic acids, a single-stranded nucleic acid that is a 5' adapter.

**[Table 34]**

| Table B | | |
|---|---|---|
| GACA | GTCA | AACT |
| ACAA | TTAT | AGGA |
| AACA | TGAA | AACG |

(2) The ligation method according to (1), wherein a ligase used for the ligation of the 3' adapter is T4 RNA Ligase 1.

(3) The ligation method according to (1) or (2), wherein each of the plurality of kinds of nucleic acids is a small RNA.

(4) A method of preparing an NGS library, including:
ligating, to a plurality of kinds of RNAs having different sequences, a 5'-adenylated single-stranded DNA that is a 3' adapter and a single-stranded nucleic acid that is a 5' adapter by the ligation method according to any one of (1) to (3);
synthesizing a cDNA by a reverse transcription reaction from the plurality of kinds of nucleic acids to which the 3' adapter and the 5' adapter have been ligated; and
amplifying the obtained cDNA by PCR.

(5) An NGS library preparation kit, including a 5'-adenylated single-stranded DNA contained in a first container, wherein a base sequence of a 5' end of the 5'-adenylated single-stranded DNA, excluding an adenylic acid at the 5' end, is any one of base sequences shown in the following Table B.

**[Table 35]**

| Table B | | |
|---|---|---|
| GACA | GTCA | AACT |
| ACAA | TTAT | AGGA |
| AACA | TGAA | AACG |

(6) The NGS library preparation kit according to (5), further including T4 RNA Ligase 1 contained in a second container.

(7) The NGS library preparation kit according to (5), further including at least one selected from the group consisting of a 5' adapter, a ligase, a reverse transcriptase, a reverse transcription primer, a PCR enzyme, and a PCR primer, in a manner in which each of these is contained in an individual container.

(8) A 3' adapter for preparing a nucleic acid library, which is a single-stranded DNA in which a nucleotide at a 5' end is adenylated, wherein a base sequence of the 5' end of the single-stranded DNA, excluding an adenylic acid at the 5' end, is any one of the base sequences shown in the following Table B.

**[Table 36]**

| Table B | | |
|---|---|---|
| GACA | GTCA | AACT |
| ACAA | TTAT | AGGA |
| AACA | TGAA | AACG |

(9) A 3' adapter for ligation, which is a single-stranded DNA in which a nucleotide at a 5' end is adenylated, wherein a base sequence of the 5' end of the single-stranded DNA, excluding an adenylic acid at the 5' end, is any one of the base sequences shown in the following Table B.

**[Table 37]**

| Table B | | |
|---|---|---|
| GACA | GTCA | AACT |
| ACAA | TTAT | AGGA |
| AACA | TGAA | AACG |

The disclosure of Japanese Patent Application No. 2023-177843 filed on October 13, 2023, and the disclosure of Japanese Patent Application No. 2024-071785 filed on April 25, 2024, are incorporated herein by reference in their entirety. All literatures, patent applications, and technical standards described herein are incorporated herein by reference to the same extent as if each individual literature, patent application, and technical standard was specifically and individually indicated to be incorporated by reference.

## Claims

1. A ligation method, comprising:
ligating, to a 3' end of each of a plurality of kinds of nucleic acids having different sequences, a 5'-adenylated single-stranded DNA that is a 3' adapter in which a base sequence of a 5' end excluding an adenylic acid at the 5' end is any one of base sequences shown in the following Table A; and
after the ligating of the 5'-adenylated single-stranded DNA, ligating, to a 5' end of each of the plurality of kinds of nucleic acids, a single-stranded nucleic acid that is a 5' adapter:
| Table A | | | | | | | |
|---|---|---|---|---|---|---|---|
| Number | Base sequence (5'→3') | Number | Base sequence (5'→3') | Number | Base sequence (5'→3') | Number | Base sequence (5'→3') |
| 1 | AAAA | 21 | GAGA | 41 | TCGA | 61 | GGAC |
| 2 | AAAC | 22 | GGAA | 42 | TCTC | 62 | GGCA |
| 3 | AAAG | 23 | GGAG | 43 | TCTT | 63 | GGCT |
| 4 | AAAT | 24 | GGGA | 44 | TGAA | 64 | GGGG |
| 5 | AACA | 25 | GTCG | 45 | TGAG | 65 | GTAG |
| 6 | AACG | 26 | TAAA | 46 | TGCA | 66 | TCAC |
| 7 | AACT | 27 | TAAC | 47 | TGGA | 67 | TGAC |
| 8 | AAGA | 28 | TAAG | 48 | TGGG | 68 | TGCG |
| 9 | AATT | 29 | TACA | 49 | TGTA | 69 | TGCT |
| 10 | ACAA | 30 | TACC | 50 | TTCT | 70 | TGGC |
| 11 | AGAA | 31 | TACG | 51 | TTTA | 71 | TTAT |
| 12 | AGAG | 32 | TAGA | 52 | TTTC | 72 | GCAA |
| 13 | AGCA | 33 | TAGG | 53 | TTTT | 73 | GCAG |
| 14 | AGCG | 34 | TATT | 54 | AACC | 74 | GTAA |
| 15 | AGCT | 35 | TCAA | 55 | AAGG | 75 | GTCA |
| 16 | AGGA | 36 | TCAG | 56 | ACTT | 76 | TCGG |
| 17 | AGGC | 37 | TCCA | 57 | AGTT | 77 | TCTA |
| 18 | AGGG | 38 | TCCC | 58 | GAAG | 78 | AGAT |
| 19 | AGTA | 39 | TCCG | 59 | GACA | | |
| 20 | GAAA | 40 | TCCT | 60 | GACC | | |

2. The ligation method according to claim 1, wherein a ligase used for the ligation of the 3' adapter and a ligase used for the ligation of the 5' adapter are each independently at least one selected from the group consisting of T4 RNA Ligase 1, T4 RNA Ligase 2, T4 DNA ligase, and a variant thereof.

3. The ligation method according to claim 1, wherein the base sequence of the 5' end of the 3' adapter is any one of base sequences shown in the following Table B:
| Table B | | |
|---|---|---|
| GACA | GTCA | AACT |
| ACAA | TTAT | AGGA |
| AACA | TGAA | AACG |

4. The ligation method according to claim 3, wherein a ligase used for the ligation of the 3' adapter is T4 RNA Ligase 1.

5. The ligation method according to claim 3, wherein each of the plurality of kinds of nucleic acids is a small RNA.

6. A method of suppressing adapter dimer formation, the method comprising:
ligating, to a 3' end of each of a plurality of kinds of nucleic acids having different sequences, a 5'-adenylated single-stranded DNA that is a 3' adapter in which a base sequence of a 5' end, excluding an adenylic acid at the 5' end, is any one of base sequences shown in the following Table A; and
after the ligating of the 5'-adenylated single-stranded DNA, ligating, to a 5' end of each of the plurality of kinds of nucleic acids, a single-stranded nucleic acid that is a 5' adapter:
| Table A | | | | | | | |
|---|---|---|---|---|---|---|---|
| Number | Base sequence (5'→3') | Number | Base sequence (5'→3') | Number | Base sequence (5'→3') | Number | Base sequence (5'→3') |
| 1 | AAAA | 21 | GAGA | 41 | TCGA | 61 | GGAC |
| 2 | AAAC | 22 | GGAA | 42 | TCTC | 62 | GGCA |
| 3 | AAAG | 23 | GGAG | 43 | TCTT | 63 | GGCT |
| 4 | AAAT | 24 | GGGA | 44 | TGAA | 64 | GGGG |
| 5 | AACA | 25 | GTCG | 45 | TGAG | 65 | GTAG |
| 6 | AACG | 26 | TAAA | 46 | TGCA | 66 | TCAC |
| 7 | AACT | 27 | TAAC | 47 | TGGA | 67 | TGAC |
| 8 | AAGA | 28 | TAAG | 48 | TGGG | 68 | TGCG |
| 9 | AATT | 29 | TACA | 49 | TGTA | 69 | TGCT |
| 10 | ACAA | 30 | TACC | 50 | TTCT | 70 | TGGC |
| 11 | AGAA | 31 | TACG | 51 | TTTA | 71 | TTAT |
| 12 | AGAG | 32 | TAGA | 52 | TTTC | 72 | GCAA |
| 13 | AGCA | 33 | TAGG | 53 | TTTT | 73 | GCAG |
| 14 | AGCG | 34 | TATT | 54 | AACC | 74 | GTAA |
| 15 | AGCT | 35 | TCAA | 55 | AAGG | 75 | GTCA |
| 16 | AGGA | 36 | TCAG | 56 | ACTT | 76 | TCGG |
| 17 | AGGC | 37 | TCCA | 57 | AGTT | 77 | TCTA |
| 18 | AGGG | 38 | TCCC | 58 | GAAG | 78 | AGAT |
| 19 | AGTA | 39 | TCCG | 59 | GACA | | |
| 20 | GAAA | 40 | TCCT | 60 | GACC | | |

7. The method of suppressing adapter dimer formation according to claim 6, wherein a ligase used for the ligation of the 3' adapter and a ligase used for the ligation of the 5' adapter are each independently at least one selected from the group consisting of T4 RNA Ligase 1, T4 RNA Ligase 2, T4 DNA ligase, and a variant thereof.

8. A method of preparing an NGS library, the method comprising:
ligating, to a plurality of kinds of nucleic acids having different sequences, a 5'-adenylated single-stranded DNA that is a 3' adapter and a single-stranded nucleic acid that is a 5' adapter by the ligation method according to any one of claims 1 to 5;
synthesizing a cDNA by a reverse transcription reaction from the plurality of kinds of nucleic acids to which the 3' adapter and the 5' adapter have been ligated; and
amplifying the obtained cDNA by PCR.

9. The method of preparing an NGS library according to claim 8, wherein the plurality of kinds of nucleic acids having different sequences are a plurality of kinds of RNAs having different sequences.

10. An NGS library preparation kit, comprising a 5'-adenylated single-stranded DNA contained in a first container, wherein a base sequence of a 5' end of the 5'-adenylated single-stranded DNA, excluding an adenylic acid at the 5' end, is any one of base sequences shown in the following Table A:
| Table A | | | | | | | |
|---|---|---|---|---|---|---|---|
| Number | Base sequence (5'→3') | Number | Base sequence (5'→3') | Number | Base sequence (5'→3') | Number | Base sequence (5'→3') |
| 1 | AAAA | 21 | GAGA | 41 | TCGA | 61 | GGAC |
| 2 | AAAC | 22 | GGAA | 42 | TCTC | 62 | GGCA |
| 3 | AAAG | 23 | GGAG | 43 | TCTT | 63 | GGCT |
| 4 | AAAT | 24 | GGGA | 44 | TGAA | 64 | GGGG |
| 5 | AACA | 25 | GTCG | 45 | TGAG | 65 | GTAG |
| 6 | AACG | 26 | TAAA | 46 | TGCA | 66 | TCAC |
| 7 | AACT | 27 | TAAC | 47 | TGGA | 67 | TGAC |
| 8 | AAGA | 28 | TAAG | 48 | TGGG | 68 | TGCG |
| 9 | AATT | 29 | TACA | 49 | TGTA | 69 | TGCT |
| 10 | ACAA | 30 | TACC | 50 | TTCT | 70 | TGGC |
| 11 | AGAA | 31 | TACG | 51 | TTTA | 71 | TTAT |
| 12 | AGAG | 32 | TAGA | 52 | TTTC | 72 | GCAA |
| 13 | AGCA | 33 | TAGG | 53 | TTTT | 73 | GCAG |
| 14 | AGCG | 34 | TATT | 54 | AACC | 74 | GTAA |
| 15 | AGCT | 35 | TCAA | 55 | AAGG | 75 | GTCA |
| 16 | AGGA | 36 | TCAG | 56 | ACTT | 76 | TCGG |
| 17 | AGGC | 37 | TCCA | 57 | AGTT | 77 | TCTA |
| 18 | AGGG | 38 | TCCC | 58 | GAAG | 78 | AGAT |
| 19 | AGTA | 39 | TCCG | 59 | GACA | | |
| 20 . | GAAA | 40 | TCCT | 60 | GACC | | |

11. The NGS library preparation kit according to claim 10, wherein the base sequence of the 5' end of the single-stranded DNA, excluding the adenylic acid at the 5' end, is any one of base sequences shown in the following Table B:
| Table B | | |
|---|---|---|
| GACA | GTCA | AACT |
| ACAA | TTAT | AGGA |
| AACA | TGAA | AACG |

12. The NGS library preparation kit according to claim 11, further comprising T4 RNA Ligase 1 contained in a second container.

13. The NGS library preparation kit according to claim 11, further comprising at least one selected from the group consisting of a 5' adapter, a ligase, a reverse transcriptase, a reverse transcription primer, a PCR enzyme, and a PCR primer, in a manner in which each of these is contained in an individual container.

14. A 3' adapter for preparing a nucleic acid library, which is a single-stranded DNA in which a nucleotide at a 5' end is adenylated, wherein a base sequence of the 5' end of the single-stranded DNA, excluding an adenylic acid at the 5' end, is any one of base sequences shown in the following Table A:
| Table A | | | | | | | |
|---|---|---|---|---|---|---|---|
| Number | Base sequence (5'→3') | Number | Base sequence (5'→3') | Number | Base sequence (5'→3') | Number | Base sequence (5'→3') |
| 1 | AAAA | 21 | GAGA | 41 | TCGA | 61 | GGAC |
| 2 | AAAC | 22 | GGAA | 42 | TCTC | 62 | GGCA |
| 3 | AAAG | 23 | GGAG | 43 | TCTT | 63 | GGCT |
| 4 | AAAT | 24 | GGGA | 44 | TGAA | 64 | GGGG |
| 5 | AACA | 25 | GTCG | 45 | TGAG | 65 | GTAG |
| 6 | AACG | 26 | TAAA | 46 | TGCA | 66 | TCAC |
| 7 | AACT | 27 | TAAC | 47 | TGGA | 67 | TGAC |
| 8 | AAGA | 28 | TAAG | 48 | TGGG | 68 | TGCG |
| 9 | AATT | 29 | TACA | 49 | TGTA | 69 | TGCT |
| 10 | ACAA | 30 | TACC | 50 | TTCT | 70 | TGGC |
| 11 | AGAA | 31 | TACG | 51 | TTTA | 71 | TTAT |
| 12 | AGAG | 32 | TAGA | 52 | TTTC | 72 | GCAA |
| 13 | AGCA | 33 | TAGG | 53 | TTTT | 73 | GCAG |
| 14 | AGCG | 34 | TATT | 54 | AACC | 74 | GTAA |
| 15 | AGCT | 35 | TCAA | 55 | AAGG | 75 | GTCA |
| 16 | AGGA | 36 | TCAG | 56 | ACTT | 76 | TCGG |
| 17 | AGGC | 37 | TCCA | 57 | AGTT | 77 | TCTA |
| 18 | AGGG | 38 | TCCC | 58 | GAAG | 78 | AGAT |
| 19 | AGTA | 39 | TCCG | 59 | GACA | | |
| 20 | GAAA | 40 | TCCT | 60 | GACC | | |

15. The 3' adapter for preparing a nucleic acid library according to claim 14, wherein the base sequence of the 5' end of the single-stranded DNA, excluding the adenylic acid at the 5' end, is any one of base sequences shown in the following Table B:
| Table B | | |
|---|---|---|
| GACA | GTCA | AACT |
| ACAA | TTAT | AGGA |
| AACA | TGAA | AACG |

16. A 3' adapter for ligation, which is a single-stranded DNA in which a nucleotide at a 5' end is adenylated, wherein a base sequence of the 5' end of the single-stranded DNA, excluding an adenylic acid at the 5' end, is any one of base sequences shown in the following Table B:
| Table B | | |
|---|---|---|
| GACA | GTCA | AACT |
| ACAA | TTAT | AGGA |
| AACA | TGAA | AACG |
